(19) Europäisches Patentamt
European Patent Office
Office européen des brevets

(11) EP 2 092 879 A1

(12) **EUROPÄISCHE PATENTANMELDUNG**
veröffentlicht nach Art. 153 Abs. 4 EPÜ

(43) Veröffentlichungstag:
**26.08.2009 Patentblatt 2009/35**

(21) Anmeldenummer: **06849626.4**

(22) Anmeldetag: **15.11.2006**

(51) Int Cl.:
***A61B 5/00*** *(2006.01)* ***A61B 5/103*** *(2006.01)*

(86) Internationale Anmeldenummer:
**PCT/RU2006/000607**

(87) Internationale Veröffentlichungsnummer:
**WO 2008/060178 (22.05.2008 Gazette 2008/21)**

(84) Benannte Vertragsstaaten:
**AT BE BG CH CY CZ DE DK EE ES FI FR GB GR HU IE IS IT LI LT LU LV MC NL PL PT RO SE SI SK TR**

(71) Anmelder: **Boot, Yuri Stanislavovich**
**Omsk 644-043 (RU)**

(72) Erfinder: **Boot, Yuri Stanislavovich**
**Omsk 644-043 (RU)**

(74) Vertreter: **Jeck, Anton et al**
**Jeck - Fleck - Herrmann**
**Patentanwälte**
**Klingengasse 2/1**
**71665 Vaihingen/Enz (DE)**

(54) **HARDWARE-SOFTWARE-AUR-UM -- VI-T-ON - GRIF TOM-SYSTEM ZUR DIAGNOSE UND KORREKTUR VON HOMÖOSTASE-STÖRUNGEN**

(57) Die Erfindung betrifft ein komplexes Gerätetechnik und Programmsystem zur Diagnose und Korrektur von Homöostasestörungen mit einem Stromimpulsgeber mit einer Schalteinheit, einer Steuereinheit mit einem Kabel zum Anschließen von drei Paaren von Hauptelektroden nämlich Stirn-, Arm- und Beinelektroden, einer Messeinheit mit einem A/D-Umsetzer und Tonbegleitung und mit einem PC mit Software (SW) zur Ausführung von drei Mess- und zwei Strombelastungsbeispielen in einer vorgegebenen Reihenfolge und graphischen Darstellungen. Damit das Gerätetechnik- und Programmsystem weit anwendbar ist und mit höher Zuverlässigkeit resultierende Kennwerte sicherstellt ohne Nebenwirkungen und Schäden des Gewebes durch Elektrolyse aufzuweisen, sieht die Erfindung vor, dass die Klangfarbe der Tonbegleitung der Messeinheit mit dem Alter und dem Geschlecht der zu untersuchenden Person verknüpft ist, dass drei zusätzliche Elektroden an bestimmten Stellen der Testperson befestigt werden und dass über eine Booster-Korrektureinheit, einem Kardiotachometer und einem Stromimpulsgeber mit Einzel- und zyklischen Impulsen eine Anzeige der Homöostase und ihre Korrektur abtastbar und anzeigbar sind.

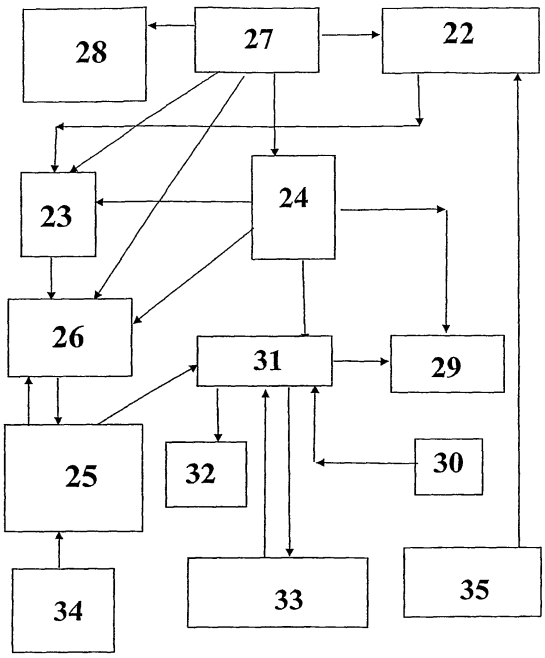


Fig. 3

EP 2 092 879 A1

## Beschreibung

Die Erfindung betrifft ein komplexes Gerätetechnik- und Programmsystem zur Diagnose und Korrektur von Homöostasestörungen mit einem Stromimpulsgeber mit einer Schalteinheit, einer Steuereinheit mit einem Kabel zum Anschließen von drei Paaren von Hauptelektroden nämlich Stirn-, Arm- und Beinelektroden, einer Messeinheit mit einem A/D-Umsetzer und Tonbegleitung und mit einem PC mit Software (SW) zur Ausführung von drei Mess- und zwei Strombelastungsbeispielen in einer vorgegebenen Reihenfolge und graphischen Darstellungen.

Die Erfindung kann in der Medizin und nämlich auf Anlagen zur Diagnose des menschlichen Gesundheitszustands eingesetzt werden. Sie kann zur Prüfung und Beurteilung der Homöostasestörungen bei Patienten sowie zur Korrektur solcher Störungen ohne Nebenwirkungen einschließlich des Stromdurchgangs durch das Gewebe eingesetzt werden. Sie kann auch zur eingehenden Diagnostik und Verkaufsbeobachtung der Korrekturergebnisse verwendet werden.

Es ist eine Einrichtung zur Erzeugung von Stromtestimpulsen mittels aufeinander folgender Leitfähigkeitsmessungen der biologisch aktiven Hautbereiche je nach sieben Ableitungen mit vorgegebener Polarität bekannt (Fig. 1).

Es handelt sich um die so genannte biofunktionelle Diagnose (BFD) von H. Pflaum und G. Janke (siehe Pflaum, H.: Praktikum der bioelektronischen Funktionen und der Regulationsdiagnostik (BFD). HF. Heidelberg. 1979. S. 1-137 [1]). Der Ablauf zur Polaritätsauswahl während der BFD ist wie folgt:

1 - Stirn links (+) - Arm links (-) \\
2 - Arm links (+) - Bein links (-);
3 - Stirn rechts (+) - Arm rechts (-) \\
4 - Arm rechts (+) - Bein rechts (-);
5 - Stirn rechts (+) - Stirn links (-) \\
6 - Arm rechts (+) - Arm links (-);
7 - Bein rechts (+) - Bein links (-).

Zahlreiche Forschungen haben nachgewiesen, dass ausgerechnet solche Polarität der BDF-Geräte die Reproduzierbarkeit für die durchgeführten Messungen bei ihrer vielfachen Anwendung bei unterschiedlichen Patienten sichergestellt hat.

Diese Geräte noch das Verfahren kamen jedoch klinisch nicht zum Einsatz. Das liegt an ihrer sehr geringen Aussagekraft und an den begrenzten Diagnosemöglichkeiten. Wenn der Strom durch das Gewebe hindurchgeht und die Polarisation vorliegt, dann ist eine Zellengefügeschädigung unvermeidlich. Diese Einrichtung weist keine therapeutischen Fähigkeiten auf.

Der nächste Stand der Technik gegenüber der angemeldeten Lösung seinem Wesen nach ist das komplexe Gerätetechnik- und Programmsystem (nachfolgend SYSTEM) zur Segmentdiagnose (siehe Unterlagen des Zentrums für intelligente Medizinsysteme "IMEDIS", Moskau; Thesen und Berichte der V. Internationalkonferenz für BRT, 1999, Teil 1, S. 380 [2]). Dieser Stand der Technik wurde als Prototyp gewählt. Dieses System umfasst, ähnlich wie die Einrichtung [1], drei Elektrodenpaare und zwar: Stirn-Stirn, Arm-Arm und Bein-Bein. Jedoch ermöglicht dieses System außer den aufeinander folgenden Leitfähigkeitsmessungen der biologisch aktiven Hautbereiche je nach sieben Ableitungen mit vorgegebener Polarität, auch Messungen mit gerade umgekehrter Polarität durchzuführen, indem sowohl die direkte als auch die Rückleitfähigkeit der Gewebe (Fig. 2) benutzt werden. Das ermöglicht es auch, die gewählten Elektrodenpaare mittels Rechteckimpulse mit Frequenz von 13 Hz mit Strom zu beanspruchen und die zusätzlichen Anpassungsreaktionen des Körpers des Untersuchten auszuwerten.

Das SYSTEM hat eine Software (SW), welche drei Mess- und zwei Strombelastungsspiele sowie ihre graphische Darstellung sicherstellt.

Die Reihenfolge der durch dieses SYSTEM vorgenommenen Segmentmessungen ist wie folgt: 8/9 - Stirn links (-/+) - Arm links (+/-) \\ 10/11 - Stirn links (-/+) - Stirn rechts (+/-) \\ 12/13 Arm rechts (-/+) - Stirn rechts (+/-) \\ 14\15 Arm rechts (-/+) - Arm links (+/-) \\ 16\17 Arm links (-/+) - Bein links (+/-) \\ 18/19 Bein rechts (-/+) - Bein links (+/-) \\ 20/21 Arm rechts (-/+) - Bein rechts (+/-) (siehe Fig. 2.) Diese Reihenfolge unterscheidet sich von den in [1] empfohlenen Messverläufen. Deswegen verfügt dieses SYSTEM über eine sehr große Streuung der resultierenden Kenngrößen.

Nach den Messungen unter Einsatz von SYSTEM [2] werden die softwaremäßig vorgegebenen Elektrodenpaare in einer festgelegten Reihenfolge (siehe Fig. 2.): 9-15-17-11-13-21-18 mit Rechteckstrom mit einer Frequenz von 13 Hz beansprucht. Diese Belastung übt auf den Körper des Untersuchten eine stimulierende Wirkung aus. Dies setzt allerdings voraus, dass Restreserven vorhanden sind. Die Stimulation mit Strom erwies sich als unerwünscht für die Anwendung bei entkräfteten und onkologischen Patienten. Das beschränkt die breite klinische Anwendung dieser Geräte.

Außerdem ergab die klinische Erprobung des SYSTEMs [2] keine 100%ige Bestätigung der vermutlichen Pathologie sogar bei ihren zuverlässigen klinischen Manifestationen. So hatten nur 4 (8,7 %) unter den 46 durch den Anmelder mittels des SYSTEMs [2] untersuchten Patienten zu unterschiedlichen Zeitpunkten nach einer Gallenblaseresektion die Krankheitszeichen des operierten Organs, das heißt, die Zeichen von Postcholezystektomiesyndrom, wobei alle Betroffenen den klinischen Verlauf von Gallendyskinesie aufwiesen.

Im SYSTEM [2] wurden Messingelektroden und vor allem rechteckige Stirnplattenelektroden verwendet. Diese Elektroden oxidierten sehr schnell und stellten keinen sicheren elektrischen Kontakt mit der Haut des Kranken sicher. Der Stirnverband presste die Kopfhaut zu stark zusammen und verursachte ausgeprägte negative Reaktionen bei den Untersuchten. Darüber hinaus bereitete die Anwendung von Beinelektroden ohne Vorwärmung im Winter große Probleme, weil das kalte Metall peripheren Gefäßekrampf verursachte und die Diagnoseergebnisse mit Fehlern (Artefakten) entstellte.

Diese Umstände ermöglichten es den Ärzten nicht, das SYSTEM [2] weit genug für ihre klinischen Untersuchungen einzusetzen.

Die bekannten Systeme DDFAO der französischen Fa. MediLD (siehe www.ddfao.ru [3]) und das Diagnose-SYSTEM AMSAT (Russland, Kovert Co., Ltd, siehe www.kovert.ru [4]) sind mit Geräten für Mikrowellentherapie kombiniert. Diese Geräte wurden für die Prüfung und Beurteilung der Homöostasestörungen entwickelt. Sie geben jedoch nicht die erforderliche hohe Sicherheit der Ausgabeergebnisse (bis 79 %) und setzen eine eingehende Schulung der Fachleute für einen sicheren Betrieb voraus.

Nach der Analyse des Stands der Technik wurden keine technischen Geräte ermittelt, welche die Screening-Diagnose der Homöostasestörungen mit einem hohen Zuverlässigkeitsgrad und ohne Nebenwirkungen sichergestellt hätten. Die Nebenwirkungen wurden dabei durch den Stromlauf durch die Gewebe zur Zeit der Messungen unter gleichzeitiger Korrektur der erkannten Abweichungen verursacht.

Die durch die angemeldete Erfindung zu lösende Aufgabe besteht darin, ein breit anwendbares komplexes Gerätetechnik- und Programmsystem für die Diagnose zum Screening und zur Beurteilung von Homöostasestörungen zu entwickeln. Dabei soll dieses Gerätetechnik- und Programmsystem einen hohen Zuverlässigkeitsgrad der resultierenden Kennwerte sicherstellen. Dieses System darf auch keine Nebenwirkungen aufweisen, welche mit den Strombeanspruchungen und der Schadwirkungen des Stroms infolge der Elektrolyse des Gewebes zusammenhängen. Das System soll auch eine gleichzeitige Korrektur der erkannten Abweichungen sowie eine eingehende Diagnostik und Verlaufsbeobachtung ermöglichen.

Die gestellte Aufgabe wird durch die Merkmale des Anspruchs 1 gelöst.

Das vorgeschlagene komplexe Gerätetechnik- und Programmsystem zur Diagnose und Korrektur der Homöostasestörungen enthält einen Stromimpulsgeber mit einer Schalteinheit und eine Steuereinheit mit Anschlusskabel für drei Paare von Hauptelektroden, nämlich Stirn-, Arm- und Beinelektroden. Das System enthält auch eine Messeinheit mit einem A/D-Umsetzer und Tonbegleitung, einen PC mit Software (SW) für die Ausführung von drei Mess- und zwei Strombelastungsspiele in vorgegebener Reihenfolge und graphischer Darstellung. In diesem System ist die Klangfarbe der Tonbegleitung der Messeinheit mit dem Alter und dem Geschlecht der zu untersuchenden Person verknüpft. An die Schalteinheit sind mindestens ein Schallsynthetisator mit einem Farbsynthetisator sowie drei zusätzliche Elektroden angeschlossen. Die Steuereinheit dient zur Steuerung von Druckmanschetten eines Norm-Tonometers. Eine der Elektroden ist die Selektivelektrode und wird am Körper der Testperson und zwar im betroffenen Bereich, das heißt in der Projektion der posttraumatischen oder Operationsnarben oder in der Projektion des Organs mit den größten Problemen (Narbe, Schmerzen u.a.m.) befestigt. Die zweite (obere) Elektrode wird in der Ebene der ersten Brustwirbel befestigt. Die dritte (untere) Elektrode wird in der Nabelhöhe befestigt. Alle Hauptelektroden sind mit einer Booster-Korrektionseinheit mit einer Spannung von 0,2 V ausgerüstet. An die Messeinheit ist ein Kardiotachometer angeschlossen. Der Kardiotachometer ist mit einer Anzeige und einem Speicher ausgerüstet. Die SW stellt zusätzlich klinische und topographisch-anatomische Nachbildung der vermutlichen Homöostasestörungen bei der Testperson sicher. Die visuelle Darstellung dieser Homöostasestörungen erfolgt in Form von graphischen Abbildungen der Stromimpulsverläufe an anatomischen Computermodellen der Hauptorgane des menschlichen Körpers. Dabei bildet der Stromimpulsgeber anstatt der Beanspruchungsimpulse die regenerierenden myostimulierenden Impulse von zwei Arten, nämlich die Einzel- und die zyklischen Impulse mit veränderbarer Frequenz, Impulsverhältnis und Amplitude. Sie werden mit den ihnen entsprechenden Tonsignalen des Schallsynthetisators und mit dem Farbenhintergrund des Farbsynthetisators synchronisiert.

Das angemeldete Gerätetechnik- und Programmsystem hat eine Urheberbezeichnung: AUR-UM--VI-T-ON ---GRIF@TOM. Diese Bezeichnung repräsentiert das Wesen der Erfindung wie folgt: G steht für graphisch, Homöostase R - regelnd, I - Interpretierungseinheit, F - Phantom, @ (A-T) A - anatomisch, T - Strom, O - Behandlung, M - von morphologischen Strukturen.

Um die optimale Funktion des angemeldeten komplexen Gerätetechnik- und Programmsystem sicherzustellen, ist es wichtig, dass jede Booster-Korrektureinheit mit der Spannung von 0,2 V an eine Eigengleichspannungsquelle angeschlossen ist. Dabei müssen alle Eigenstromquellen galvanisch getrennt sein, einen hohen Ausgangswiderstand aufweisen, sich vor dem Diagnoseanfang einschalten und die vorgegebene Polarität haben.

Für die optimale Funktion des angemeldeten Gerätetechnik- und Programmsystems ist es auch wichtig, dass die Elektroden mit einer Thermofixiereinheit versehen und aus einem unmagnetischen und unoxydierbaren Metall gefertigt sind. Die Stirnelektroden müssen auf einem flexiblen Ring angeordnet sein. Der Ring stellt ein regelbares Anpressen an die Stirnhaut während der Diagnose sicher. Die Arm- und Beinelektroden sind mit Einschnitten zum Schweißabfluss aus dem Elektrodenraum versehen. Diese Elektroden sind an individuellen dielektrischen Untersätzen arretiert. Die zusätzlichen Elektroden sind in Form von einem Sauggreifer oder aus einem flexiblen leitfähigen Gummi gefertigt. Die zusätzlichen Elektroden werden am Körper der Testperson mittels der Kleidung (Hemdkragen, Bauchgürtel) befestigt.

Nachfolgend wird das Wesen der angemeldeten Erfindung anhand von graphischen Materialien erläutert.

Es zeigen:

Fig. 1 die Reihenfolge und Polarität von sieben klassischen Messungen während der Durchführung einer biofunktionellen Diagnose gemäß BFD [1]: 1 - Stirn links (+) - Arm links (-) \\ 2 - Arm links (+) - Bein links (-); 3 - Stirn rechts (+) - Arm rechts (-) \\ 4 - Arm rechts (+) - Bein rechts (-); 5 - Stirn rechts (+) - Stirn links (-) \\ 6 - Arm rechts (+) - Arm links (-); 7 - Bein rechts (+) - Bein links (-).

Fig. 2 die Reihenfolge und Polarität der Ableitungen bei der Durchführung von Messungen am SYSTEM [2] - IMEDIS: 8/9 - Stirn links (-/+) - Arm links (+/-) \\ 10/11 - Stirn links (- /+) - Stirn rechts (+/-) \\ 12/13 Arm rechts (-/+) - Stirn rechts (+/-) \\ 14\15 Arm rechts (-/+) - Arm links (+/-) \\ 16\17 Arm links (-/+) - Bein links (+/-) \\ 18/19 Bein rechts (-/+) - Bein links (+/-) \\ 20/21 Arm rechts (-/+) - Bein rechts (+/-) und von Belastungstests: 9-15-17-11-13-21-18 mit Rechteckstrom mit einer Frequenz von 13 Hz.

Fig. 3 das Blockschaltbild des Gerätetechnik- und Programmsystem AUR-UM--VI-T-ON ---- GRIF@TOM gemäß der angemeldeten Erfindung, einschließlich:
22 Stromimpulsgeber (Generator für Gleich- und Impulsstrom mit veränderbarer Frequenz, Tastverhältnis und Amplitude,
23 die Schalteinheit, welche die Reihenfolge und den softwaregestützten Anschluss des vorgegebenen Elektrodenpaar sicherstellt,
24 die Steuereinheit, welche die Anschlussmodi des Arbeitselektrodenpaars an die Messeinheit oder an den Kardiotachometer oder an den internen (22) bzw. externen (25) Stromimpulsgeber sicherstellt, um Messungen oder Heilkorrekturen durchzuführen,
25 die Arbeitselektrodenpaare mit einer Thermostabilisierungseinheit. Als Thermostabilisierungseinheit wird eine medizinische Wärmflasche benutzt: Die Hauptelektroden umfassen die Stirnelektroden Nr. 1, 2, Armelektroden Nr. 3, 4, Beinelektroden Nr. 5, 6; zusätzliche Elektroden - Selektivelektrode Nr. 7, obere Elektrode Nr. 8 und untere Elektrode Nr. 9;
26 eine Messeinheit mit dem A/D-Umsetzer (Analog-Digitalumsetzer), der die Daten über die Testperson an die PC-Software zur Analyse und visuellen Darstellung überträgt,
27 einen PC komplett mit Monitor, Drucker, Tastatur und Maus,
28 einen Schallsynthetisator, z. B. Haushalts-Stereokopfhörer und einen Farbsynthetisator, z. B. ein medizinisches Gerät für Farbtherapie GESKA-POLITSVET,
29 eine Steuereinheit zur Steuerung von Druckmanschetten und ihren Kombinationen beim Betrieb: eine Armdruckmanschette, zwei Armdruckmanschetten, eine Beindruckmanschette, zwei Beindruckmanschetten, eine Arm- und eine Beindruckmanschette rechts mit einem Hand- oder elektrischen Kompressor; die Druckmanschetten gehören zu dem medizinischen Standardtonometer zur Blutdruckmessung,
30 Ohrklipps zum Betrieb eines Kardiotachometer,
31 eine Kardiotachometereinheit (Herzrhythmusparametermesser),
32 eine Anzeigeeinheit des Kardiotachometers zur Anzeige des Herzrhythmus und seiner Frequenz (Bradykardie und Tachykardie),
33 eine Einheit zur Aufnahme und Wiedergabe der Herzfrequenzkennwerte, um diese ununterbrochen aus dem Speicher in die Anzeigeeinheit des Kardiotachometers während der Messungen und der Heilzyklen auszugeben,
34 eine Booster-Korrektureinheit,
35 einen externen medizinischen physiotherapeutischen Strompulsgeber, z. B. DiaDENS-T.

Fig. 4 das Aussehen eines Grundgehäuses der angemeldeten Anlage Gerätetechnik- und Programmsystems, Front- und Rückwandplatte des Grundgehäuses.

Fig. 5 das Aussehen der Peripherieeinrichtungen des Gerätetechnik- und Programmsystems, nämlich des Anzeigers 32 und des Ohrklipps 30.

Fig. 6 die Stirnelektroden Nr. 1 und 2 des angemeldeten Gerätetechnik- und Programmsystems auf einem elastischen Fixierelement.

Fig. 7 die Armelektroden Nr. 3 und 4 des angemeldeten Gerätetechnik- und Programmsystems auf einem dielektrischen (Holz-) Untersetzer.

Fig. 8 die Beinelektroden Nr. 5 und 6 auf einem dielektrischen (Holz-) Untersetzer mit elektrischer Heizung mittels einer medizinischen Wärmflasche.

Fig. 9 die zusätzlichen Elektroden Nr. 7, 8 und 9 in Form von einem Sauggreifer oder flexible Plattenelektroden aus leitfähigem Gummi.

Fig. 10 ein medizinisches physiotherapeutisches Gerät DIA-DENS-T, welches an das angemeldete Gerätetechnik- und Programmsystem zwecks allgemeiner (für alle Ableitungen) oder selektiver (für das ausgewählte Elektrodenpaar) Heilwirkung.

Fig. 11 die Reihenfolge 36 - 65 zum Anschluss der Messeinheit des angemeldeten Gerätetechnik- und Programmsystems unter Berücksichtigung der Polarität: Für 6 Elektroden (15 Ableitungen): SL - Stirn links - 1\ AL - Arm links - 2\ BL - Bein links - 3\ BR - Bein rechts - 4\ AR - Arm rechts - 5\ SR - Stirn rechts - 6 ---- 1-6\ 2-6\ 1-5\ 2-1 \ 6-5\ 2-5\ 6-3\ 1-4\ 3-1 \ 6-4\ 3-5\ 2-4\ 3-2\ 5-4\ 3-4.

Fig. 12 die Reihenfolge 36 - 77 zum Anschluss der Messeinheit des angemeldeten Gerätetechnik- und Programmsystems unter Berücksichtigung der Polarität: Für 7 Elektroden (15+6=21 Ableitungen): 1-6\ 2-6\ 1-5\ 2-1 \ 6-5\ 2-5\\ 7-1 \ 7-6\ 7-2\ 7-5\ 7-3\ 7-4\\ 6-3\ 1-4\ 3-1 \ 6-4\ 3-5\ 2-4\ 3-2\ 5-4\ 3-4.

Fig. 13 die Reihenfolge 36 - 65, 78 - 103 zum Anschluss der Messeinheit des angemeldeten Gerätetechnik- und Programmsystems unter Berücksichtigung der Polarität: Für 8 Elektroden (15+13=28 Ableitungen) 1-6\ 2-6\ 1-5\\ 6-8\ 8-1 \\ 2-1 \ 6-5\ 2-5\\8-5\ 2-8\ 8-4\ 3-8\\ 6-3\ 1-4\ 3-1 \ 6-4\\9-1 \ 6-9\ 9-5\ 3-9\\ 3-5\\ 9-4\ 2-9\\ 2-4\ 3-2\ 5-4\ 3-4.

Fig. 14 die Reihenfolge 36 - 107 zum Anschluss der Messeinheit des angemeldeten Gerätetechnik- und Programmsystems unter Berücksichtigung der Polarität: Für 9 Elektroden mit einer Maximalanzahl von Ableitungen (15+13+8=36 Ableitungen) 1-6\ 2-6\ 1-5\\ 6-8\ 8-1\\ 2-1 \ 6-5\ 2-5\\ 8-5\ 2-8\ 8-4\ 3-8\\ 7-17-6\ 7- 2\ 7-5\ 7-8\ 7-9\ 7-3\ 7-4\\ 6-3\ 1-4\ 3-1 \ 6-4\\ 9-1\ 6-9\ 9-7\ 9-5\ 3-9\\ 3-5\\ 9-4\ 2-9\\ 2-4\ 3-2\ 5-4\ 3-4.
Die Untersuchungen wurden an einem Patienten mit einer Operationsnarbe nach Appendektomie (Wurmfortsatzentfernung) vorgenommen.
Die Elektrode Nr. 7 wurde in die rechte Krummdarm-Leistengegend des Patienten in der Projektion der Operationshautnarbe gesetzt.

Fig. 15 Polarität und Reihenfolge zum Anschluss der Hauptelektroden zwecks Heilung mit Korrekturboosterspannung: 108 - BL - SL, 109 -SR-BR, 110-SL-SR, 111 -AL-AR, 112-BL-BR, 113-SR -AR, 114-AL-SL, 115-AL-BR, 116-BL-AR.

Fig. 16 die Versionen der dynamischen Kennlinien beim Stromfluss über das für die Messungen angeschlossene Elektrodenpaar: 117 - Kennwerte innerhalb der Normspannweite von 72-86 KE (konventionellen Einheiten), nebeneinander laufende waagerechte Linien:
118 die zweite Messung weist einen fallenden Verlauf, nämlich Instabilitätsphänomen auf; das ist das Kennzeichen der eingeschränkten Anpassungsreserven,
119 Phänomen der Kennwertedissymmetrie - die Kreuze - wird bei Patienten mit angeborener Pathologie, Narben, Fibrose, Onkogenese und bei Helmintheninvasion (besonders bei Kindern) festgestellt,
120 Kennwerte von beiden Messungen sind außerhalb der Norm und unterscheiden sich sehr voneinander; es handelt sich um das Phänomen von Dissoziation oder Deviation, Halbleiterverhalten der Gewebe, das Kennzeichen der ausgeprägten Homotoxikose,
121 Kennwerte von beiden Messungen sind höher als die Normspannweite; es handelt sich um eine Polarisationserscheinung. Diese Polarisationserscheinung ist für Anpassungsreaktionen, akute Entzündungsprozesse, Gewebeödem eigen. Sie entspricht den feuchten Hautdecken, der mechanisch beschädigten Haut und der OH-Hydration,

122 Kennwerte von beiden Messungen liegen unterhalb der Normspannweite; es handelt sich dabei um eine Depolarisationserscheinung. Sie ist der Hypotonie, der Anämie, der Fibrose, der Sklerose, Dystonie, den chronischen Erkrankungen, der Onkogenese, der Trockenheit und Verdickung der Hautdecken, der H+Dehydratation eigen.

Fig. 17 und 18 Auslegungsvarianten bei Wirbelsäuleproblemen (eventuelle Erkrankungen, Traumata und durchgemachte oder erwartete Operationen bei der zu untersuchenden Person in Übereinstimmung mit Target-Organen) oder Zahnproblemen (eventuelle Kopplungsorgane).

Fig. 19 und 20 Zonen der +\- Polarisation (H+Dehydratation und OH-Hydratation); sie sind dementsprechend den chronischen oder akuten Pathologievorgängen eigen.

Fig. 21 Varianten der graphischen Auslegung der erkannten Homöostasestörungen, d. h. der Homotoxikosezonen - 123 (Dermatom), vor - 124 und nach - 125 der Galvanokorrektur. Die Galvanokorrektur wurde in diesem Fall in Monobetrieb, nämlich mittels der selektiven Korrekturimpulsgabe bei "schlechtester Ableitung" vorgenommen.

Die Homotoxikosebereiche (Stadien) wurden nach dem Urheberverfahren ermittelt:

1 hellgelbe Farbe oder ein Zeichen - Doppelkreis mit Standardgröße (4 - 5 mm): Wenn beide Linien innerhalb des Bereichs von 72 - 86 KE nebeneinander liegen, beträgt das Delta max. 3 KE, so liegt kein Abfall vor.

2 dunkelgelbe Farbe oder ein Kreis mit einer Umrandung: Beide Linien liegen innerhalb des Bereichs von 72 - 86 KE mit einer Divergenz von 3 bis 8 KE, eine Linie ist innerhalb dieses Bereichs, die andere Linie liegt um 2 KE höher oder tiefer, alle Linien weisen Abweichungen von der Waagerechten bis 3 KE auf.

3 orange Farbe oder ein Achteck, wenn mindestens eine Linie um 8 KE tiefer und um 15 KE höher als 72 KE liegt; das Delta beträgt 8 bis 14 KE; mindestens eine Linie hat eine Abweichung bis zu 5 KE von der Waagerechten.

3-4 (4) orange Farbe oder ein größeres Achteck (7-8 mm); wenn beide Linien um 8 KE tiefer und um bis 15 KE höher als 72 KE liegen; das Delta beträgt 8 bis 14 KE; beide Linien weisen eine Abweichung bis 8 KE von der Waagerechten auf oder mindestens eine Linie weist eine Abweichung bis 10 KE von der Waagerechten auf.

4-3 (5) rote Farbe oder ein größeres Sechseck (7-8 mm); das Delta beträgt 14 bis 18 KE, wenn mindestens eine Linie um 15 KE tiefer und bis zu 25 KE höher als 72 KE liegt, eine Linie ist höher als 88 bis 92 KE; die Waagerechte wird durch mindestens eine Linie um bis zu 12 KE nicht eingehalten.

4- (6) rote Farbe oder ein Sechseck mit Standardgröße; das Delta beträgt 14 bis 18 KE; wenn beide Linien um 15 KE tiefer und um 25 KE höher als 72 KE liegen; eine oder beide Linien liegen höher als 88 bis zu 92 KE; die Waagerechte wird durch beide Linien um bis zu 8 KE und durch mindestens eine Linie um bis zu 12 KE nicht eingehalten.

5- (7) dunkelrote Farbe oder ein Quadrat; das Delta beträgt 18 bis 22 KE;wenn beide Linien um 25 KE tiefer und bis zu 35 KE höher als 72 KE liegen; eine oder beide Linien liegen höher als 92 KE; die Waagerechte wird durch beide Linien um bis zu 12 KE und durch mindestens eine Linie um bis zu 15 KE nicht eingehalten.

6- (8) schwarze Farbe oder ein Dreieck: Wenn mindestens eine Kreuzung der Linien vorliegt; wenn beide Linien um 35 KE und mehr tiefer als 72 KE liegen; das Delta beträgt mehr als 22 KE; die Waagerechte wird durch beide Linien um bis 15 KE und durch mindestens eine Linie um bis zu 18 KE nicht eingehalten.

9 ein schwarzes Rechteck: mehr als 3 Kreuzungen bei unterschiedlichen Ableitungen, wenn beide Linien um 35 KE und mehr tiefer als 72 KE liegen; mindestens 4 Ableitungen; das Delta beträgt mehr als 22 KE; die Waagerechte wird durch beide Linien um bis 18 KE und mehr KE nicht eingehalten.

Fig. 22 die Pentagramm-Variante der Galvanokorrektur: Stirn rechts (-) - Stirn links, Arm links, Arm rechts, Bein rechts, Bein links (+, +, +, +, +).

Fig. 23 alle 32 möglichen Pentagrammverhalten der Galvanokorrektur.

Fig. 24 das bimodale 126-Pentagrammverfahren der Galvanokorrektur (8-Ableitungen), das Tripolar- 127 und das Tetrapolarverfahren 128 der Galvanokorrektur.

Fig. 25 die Version der NANO-(9) Frequenz-Galvanokorrektur: 129 - aus dem Hochfrequenzbereich (1-2\ 2-6\ 6-5\ 5-1\ 1-3\ 3-6\ 6-4\ 4-1\ 1-6), 130 - aus dem Mittelfrequenzbereich (2-1\ 1-5\ 5-6\ 6-1\ 2-3\ 3-5\ 5-4\ 4-2\ 2-5) , 131 - aus dem Niederfrequenzbereich (3-2\ 2-4\ 4-5\ 5-3\ 3-1 \ 1-4\ 4-6\ 6-3\ 3-4).

Fig. 26 eine Version der Ausgleichsbreiband-Galvanokorrektur (hinauf-hinab) für 15 Ableitungen: 5-4\ 3-2\\ 3-5\ 2-4\\ 3-6\ 4-1\ 3-1\ 6-4\\ 2-6\ 1-5\ 2-1 \ 6-5\\ 1-6\ 2-5\ 3-4).

Fig. 27 das dynamische Ergebnis der durchgeführten Messungen in Form von Kreisdiagrammen: Abweichungen der ersten und der zweiten Messung des 1, 2 und 3 Messzyklus und der Kennwertinstabilität.

Fig. 28 die resultierenden Angaben in dem vom Urheber vorgeschlagenen Modus -CHROMOKINETIK.

Fig. 29 die resultierenden Angaben in dem vom Urheber vorgeschlagenen Modus - Gesamtergebnisse aller drei Messungen.

Fig. 30 die resultierenden Angaben in dem vom Urheber vorgeschlagenen Modus - Asymmetrie der Gesamtangaben für den oberen und unteren sowie für den rechten und den linken Körperteil der zu untersuchenden Person.

Fig. 31 die resultierenden Angaben in dem vom Urheber vorgeschlagenen Modus - dynamische Messungen; es werden die besten und die schlimmsten Kennwerte von allen gemessenen Ableitungen unter Berücksichtigung ihrer Polarität abgebildet.

Fig. 32 die ausgedruckten Endergebnisse mit Integralkennwerten und der Liste der "schlimmsten Bereiche".

Das in Fig. 3 angeführte Schaltbild des angemeldeten Gerätetechnik- und Programmsystems enthält einen Stromimpulsgeber 22 mit einer Schalteinheit 23, eine Steuereinheit 24 und Anschlusskabel für drei Paare von Hauptelektroden 25: Stirn-, Arm- und Beinelektroden mit einem Thermostabilisator, eine Messeinheit 26 mit einem A/D-Umsetzer und einen PC 27 mit Software (SW) zur Ausführung von drei Messzyklen (Vorwärts- und Rückwärtszyklen) und zwei Stabilisationsstromzyklen (Einzel- und Gruppenzyklen) in vorgegebener Reihenfolge mit graphischer und Tonbegleitung.

Um die Zuverlässigkeit der ermittelten Kennwerte zu erhöhen, werden alle Elektroden 25 vor dem Anlegen auf der zu untersuchenden Person einer Thermofixierung ausgesetzt (d. h., sie werden bis 37 Grad. mittels einer medizinischen Wärmflasche erwärmt). Sie werden aus einem unmagnetischen und unoxydierbaren Material gefertigt, z. B. aus Titan oder Medizinstahl.

Die Stirnelektroden 1 und 2 (siehe Fig. 6) werden mit einem Durchmesser von 23,0 mm gefertigt. Ihre Arbeitsfläche wird geschliffen. Die Stirnelektroden 1 und 2 werden mit einem Abstand von 72,3 mm voneinander auf einem flexiblen Reifen angeordnet. Der Reifen stellt ein regelbares Anpressen an die Stirnhaut beim Anlegen sicher.

Die Armelektroden 3 und 4 (siehe Fig. 7) werden flach gefertigt und sind mit Einschnitten zum Abfluss von Schweiß aus dem Elektrodenraum versehen. Die Armelektroden 3 und 4 werden auf einem dielektrischen Untersetzer befestigt, um Zwischenelektrodenschlüsse zu vermeiden.

Die Beinelektroden 5 und 6 (siehe Fig. 8) werden ebenfalls mit Einschnitten versehen. Sie werden auf einem dielektrischen Untersetzer befestigt und sind mit einer Einheit für Dauerthermofixierung ausgerüstet. Als ein solcher Thermostabilisator wird z. B. eine genormte medizinische Wärmflasche angewendet (TU 3468-001-0001578-2004), z. B. 3M-01-7, Bryansk.

Die zusätzlichen Elektroden werden in Form von einem Sauggreifer oder flexiblen Plattenelektroden aus leitfähigem Gummi (siehe Fig. 9) eingesetzt. Dabei werden für Ihre Arretierung auf dem Körper der zu untersuchenden Person der Kragen bzw. der Kleidungsgurt bzw. externe elastische Riemen benutzt.

Nachdem die Vorwärts- und Rückwärtszyklen der Messstromimpulse des ersten Messzyklus abgeschlossen sind, wird der Stromimpulsgeber 22 in den Homöostase-Korrekturbetrieb geschaltet. Dieser Betrieb dient zur Regeneration der Gewebe mittels einzelner myostimulierender Impulse. Dabei wird die kleinste Heilamplitude des Anfangsignals (die so genannte Anpassungszeit) gewählt. Diese Amplitude wird zur Endphase der Korrektur hin allmählich vergrößert. Nach dem zweiten Messzyklus werden die Gewebe mittels der Einwirkung von myostimulierenden Impulspaketen (von je 10 bis 40 Impulse je nach Frequenz) regeneriert. Dabei wird die maximale Heilamplitude des Anfangsignals gewählt. Diese Heilamplitude darf allerdings 2,5 V nicht überschreiten. Die Heilamplitude wird allmählich zur Endphase der Korrektur hin reduziert und zwar bis die unterschwelligen Informationsgrößen in Höhe von 0,1 V erreicht sind. Die Korrekturstromimpulse kommen mit veränderbarer Frequenz, Tastverhältnis und Amplitude und synchronisieren mit ihren entsprechenden Tonsignalen und dem Betrieb des Ton-Farbsynthetisators 28.

Die Reihenfolge und die Polarität des Verlaufs der Strommess- und Korrekturimpulse werden je nach der Anordnung der Elektroden auf dem Körper der zu untersuchenden Person festgelegt und hängen von der Anzahl der benutzten Arbeitselektroden ab: zwei, vier, sechs, 6+1 (sieben), 6+2 (acht) oder 6+2+1 (neun) (siehe Fig. 11 - 14). Gerade diese zusätzlichen Messungen erhöhen die Zuverlässigkeit der durchgeführten Forschungen.

Im angemeldeten komplexen Gerätetechnik- und Programmsystem werden zwei und vier Elektroden zum Fol-Test als Bestandteil des Screeningverfahrens angewendet. Hier werden sechs traditionelle Standardhauptelektroden eingesetzt: Zwei Stirn-, zwei Arm- und zwei Beinelektroden. Diese Elektroden werden an die Messschaltung 30 Mal in einer strikt vorgegebenen Reihenfolge und Polarität über 15 Ableitungen (siehe Fig. 11) angeschlossen.

Die Differentialdiagnose bedarf des Anschlusses von noch einer selektiven Elektrode. Diese zusätzliche (siebte) Elektrode wird an der Haut in der Projektion der posttraumatischen oder der Operationsnarbe oder in der Projektion des Organs mit den größten Problemen (z. B. über der Leber, falls die untersuchte Person sich über die Schmerzen im rechten Hypochondrium beschwert) angeordnet. Dabei nimmt die Anzahl der Ableitungen bis 21 und die Anzahl der Messungen bis 42 (siehe Fig. 12) zu.

Zur eingehenden Diagnostik werden die sechs Hauptelektroden durch zwei zusätzliche Elektroden ergänzt, welche in Form einer Sauggreifer-Arretierung oder aus leitfähigem Gummi gefertigt sind, und zwar: die obere (die achte) - Vorderelektrode für Frauen und Hinterelektrode für Männer, die untere (die neunte) - Vorderelektrode für Männer und Hinterelektrode für Frauen (siehe Fig. 13). Dabei wird die Anzahl der möglichen Ableitungen bis 15+13=28 erhöht, und die Anzahl der Messungen der Vorwärts- und Rückwärtsleitfähigkeit der Gewebe nimmt bis 56 zu.

Zur vollständigen Diagnose und Korrektur der Homöostasestörungen werden sechs (1 - 6) Haupt- und drei zusätzliche (7, 8, 9) Elektroden (siehe Fig. 14) angeschlossen. Dabei wird die Anzahl der eventuellen Ableitungen bis 15+13+8=36 erhöht, und die Anzahl der Messungen der Vorwärts- und Rückwärtsleitfähigkeit der Gewebe steigt bis 72. Solche Messungen zwischen allen neun Elektroden wurden vom Urheber in allen möglichen Kombinationen POLYDIAGONAL genannt.

Die zusätzlichen Prüfungen zur selektiven Auswertung der Mikrozirkulationsstörungen werden vor und nach dem Anlegen von Arm- und/oder Beindruckmanschetten 29 vom Standardtonometer durchgeführt.

Die Kopf-, Arm-, Stirn- und zusätzlichen Elektroden vom Gerätetechnik- und Programmsystem übertragen Gleichstrom an die Haut der zu untersuchenden Person für galvanische Messungen an der Haut. Das sind die 1., 3., 5. Stufen des Diagnosezyklus und die myostimulierenden (neurotrope) Impulse zur Homöostasekorrektur und für Heilzwecke. Anstatt von rechteckigen Belastungsimpulsen (aus dem Stand der Technik) werden Einzelimpulse (2. Zyklus) und Impulspakete à 10 - 20 Spitzen je nach der benutzten Frequenz (4. Zyklus) mit variabler Amplitude von 0 bis 2,5 Volt, der Frequenz von 0,3 bis 100,0 Hz und dem Impulsschaltverhältnis von 6 % bis 99 % benutzt. Die Messungen des hautgalvanischen Gleichstromwiderstands zwischen den angelegten Elektroden werden in allen möglichen Ableitungskombinationen durchgeführt. Allerdings wird immer die vorgegebene Reihenfolge eingehalten und nie geändert (siehe Fig. 11 - 14). Diese Tatsache ermöglicht es, die Datenbanken für die Vergleichsanalyse und die nachfolgende Verifizierung der ermittelten Daten zu erstellen. Es kann auch als Lernphänomen des angemeldeten Diagnosesystems benutzt werden, indem dieses System als ein Expertenabfragesystem wirkt.

Werden bei der zu untersuchenden Person ausgeprägte Homöostasestörungen erkannt, so wird ein zusätzlicher Heilmodus GALVANOPHORESE eingeschaltet. Dieser Modus korrigiert die Strompolarität auf den Elektroden, die sich in Problemzonen befinden (siehe Fig. 21 - 26 und Tabelle Nr. 1 - 5).

Die Elektroden des komplexen Gerätetechnik- und Programmsystems ermöglichen die selektive Einwirkung mittels Heilimpulse des medizinischen Geräts vom Typ Dia-DENS-T, welches als ein externer Generator angeschlossen wird (siehe Fig. 10, Registrierschein des medizinischen Geräts Nr. 29/23020502/3939-02 vom 18. Juni 2002, ausgestellt durch Ministerium für Gesundheitswesen der RF [3]). Es können dafür auch ein unabhängiges Elektrostimulationsgerät AES ZhKT (

АЭС ЖКТ,

NIIPP Tomsk, TU Nr. 9444-017-07543077-2004) und andere medizinische physiotherapeutische Geräte verwendet werden. Es wird eine Komplextherapie verordnet. Die Patienten werden einen Monat später zur erneuten Untersuchung und zum Vergleich der Kennwerte mit den vorigen Messergebnissen zwecks Verlaufsbeobachtung des Erneuerungsprozesses der Homöostase aufgefordert.

Um die Zuverlässigkeit der Untersuchung zu erhöhen, wird ein Ohrklipps 30 an die Messeinheit 26 mit einem A/D-Umsetzer (A/D-Umsetzer) angeschlossen und mit dem Kardiotachometer 31 (siehe Fig. 3, 5) verbunden. Die Aufnahme des Elektrokardiogramms, d. h. der Herzfrequenz, der zu untersuchenden Person erfolgt nur in den Pausen zwischen den Mess- und Korrekturstromimpulsen durch die Armelektroden und den Ohrklipps. Diese Daten werden auf dem Anzeiger 32 visuell dargestellt. Dabei wird der aktuelle Rhythmus von EKG im Speicher der Einheit 33 zur Aufnahme, Wiedergabe und Synchronisation des kardiologischen Signals aufgenommen. Dieser EKG-Rhythmus wird während der Pausen zwischen den Messungen aktualisiert und während der Messung aus dem Speicher auf dem Anzeiger 32 mit der das Herzklopfen nachbildenden Tonbegleitung gleichzeitig mit Tonbegleitung der Messeinheit wiedergegeben. Es wurde experimentell bewiesen, dass diese zusätzlichen Funktionen des Kardiotachometers 31 und die Synchronisation des Anfangs der Messimpulse mit dem kardiologischen Eigenrhythmus, welcher durch den Kardiotachometer 31 ausgesondert wurde, der Ausbildung zu einer festen biologischen Rückverbindung beitragen. Außerdem stellen sie einen hohen Zuverlässigkeitsgrad der ermittelten Kennwerte ohne Nebenwirkungen sicher. Diese Nebenwirkungen hängen normalerweise mit den minimalen Strommesszyklen zusammen.

Es wird auch dadurch gefördert, dass die Klangfarbe der Tonbegleitung der Messeinheit 26 softwaregestützt gemäß einer Formel je nach Alter und Geschlecht der zu untersuchenden Person berechnet wird: $F = 440 - W * k$ (Hz). Dabei sind: F die Tongeneratorfrequenz, W das Alter der zu untersuchenden Person, k ein Vervielfachungsfaktor, welcher für Männer 2,741 und für Frauen 2,120 beträgt.

Um die zu untersuchende Person vorzubereiten, wird vor dem Anfang des 1. Messzyklus eine Gleichspannung von 0,2 V mit vorgegebener Polarität an die Hauptelektroden angelegt. Diese Gleichspannung kommt von getrennten Referenzspannungsquellen, nämlich der Gleichstrom-Booster-Korrektureinheit 34. Diese Spannungsquellen sind galvanisch getrennt und weisen einen hohen Ausgangswiderstand auf.

Die Software 27 des Gerätetechnik- und Programmsystems stellt eine zusätzlich klinische und topographisch-anatomische Simulation der vermutlichen Homöostasestörungen bei der zu untersuchenden Person sicher. Die Visualisierung erfolgt in graphischer Form zur Darstellung des dynamischen Verlaufs der Stromimpulse an anatomischen Computersimulationsmodellen der Körperoberfläche der zu untersuchenden Person (Dermatom), der Wirbelsäulensegmente, der Zahnmodellen sowie der menschlichen Hauptkörperorgane.

Die Programmeinheit 27 ermöglicht es, noch vor Beginn der Untersuchung eine individuelle klinische und topographisch-anatomische Simulation von möglichen Störungen im Körper der zu untersuchenden Person vorzunehmen.

Das Wesen der Methode besteht im Folgenden. Vor der Aufnahme befragt der Bediener eingehend die zu untersuchende Person über die früheren Verletzungen und Operationen, wertet ihr Aussehen, die Beschwerden, vermutliche oder bereits gestellte Diagnose und die bereits vorhandenen Angaben von anderen Untersuchungsverfahren aus, z. B. die Laborangaben über Blut- oder Urinanalyse. Diese Angaben werden in Form von erwarteten Störungen in die Datenbank für den jeweiligen Patienten eingegeben. Danach werden diese Angaben unter Einsatz von durch die Elektroden ermittelten Kennlinien präzisiert und visualisiert, ausgelegt und korrigiert, indem das Phänomen der biologischen Rückverbindung angewendet wird.

Zum Beispiel: Der Patient teilt mit, dass seine Gallenblase im Zusammenhang mit Gallensteinleiden entfernt wurde. Das Programm visualisiert automatisch Leberprobleme und wertet die möglichen Ursachen der Steinbildung bei dieser zu untersuchenden Person unter Berücksichtigung ihrer spezifischen Kennwerte aus. Die durch den Anmelder vorgeschlagene individuelle Simulation erhöht wesentlich die Zuverlässigkeit der durchgeführten Diagnoseprüfungen und der Modi der Homöostasekorrektur.

Über einen Rechner sowie eine SW synchronisiert das Gerätetechnik- und Programmsystem die den Wechsel von Strom-, Ton- und Farbreizfaktoren unter Einsatz von einer handelsüblichen Soundkarte, Lautsprecherboxen bzw. Kopfhörer und einem Farbmonitor.

Das System bekommt den Herzkontraktionsimpuls der zu untersuchenden Person übermittelt durch die Handelektroden 25 Nr. 3 und 4 und den Ohrklipps 30. Die Kennwerte dieser Person werden auf einem Anzeiger 32 innerhalb des Bereichs von 40 - 120 Schläge pro Minute visuell dargestellt und in Bezug auf Normgrößen (Kardiotachometer 31) ausgewertet. Damit wird die Zuverlässigkeit der Untersuchung erhöht und der Emulationsbetrieb (die Untersuchung ohne Anwesenheit des Patienten) ausgeschlossen.

Anhand des Ohrklipps 30 überwacht das System den Anschluss der zu untersuchenden Person an das Gerätetechnik- und Programmsystem 27 sowie das Vorhandensein eines kardiologischen Signals an den Armelektroden und gleicht die Industriestörungen an den Elektroden aus.

Das System überwacht die Lage und den Zustand aller Elektroden 25 Nr. 1 - 9 und zeigt diese mittels einer Farbänderung der LED-Anzeiger und mittels eines Tonsignals an, wenn die Elektroden sich nicht an der Haut des Patienten befinden oder geschlossen sind.

Anhand einer eigenen Original-Testsoftware überwacht das System die richtige Anordnung und die Polarität beim Anschluss der für die Messungen und die Korrekturzyklen benutzten Elektroden auf dem Körper der zu untersuchenden Person.

Anhand von einem Tonsignal zeigt das System die Kennwerte der Mess- und Korrekturstromimpulse an. Bei geringen Kenngrößen sowie bei alten Personen klingt ein tieferes Tonsignal. Bei hohen Kenngrößen sowie bei Frauen und Kindern steigt die Klangfarbe an.

Zur Synchronisation des kardiologischen Signals mit Mess- und Heilimpulsen hat der Erfinder ein Standardtonometer 29 (siehe Fig. 3) eingesetzt. Dieser Standardtonometer 29 wurde mit einer, zwei (Oberarm oder Oberschenkel rechts und links), drei oder mit vier Manschetten auf einmal (Oberarme + Oberschenkel) betrieben. Die Manschetten werden mittels eines gemeinsamen Kompressors manuell oder automatisch per Steuerimpuls vom Programm gepumpt. Dies erfolgt zuerst vor dem Entstehen der Korotkov-Töne (die Venen sind vollständig zusammengedrückt) und danach vor dem Verschwinden der Korotkov-Töne (die Arterien sind vollständig zusammengedrückt). Danach wurden die Manschetten entlüftet. Die Messungen wurden vor und nach dem Zusammendrücken der Blutgefäße mit Manschetten vorgenommen. Dieser Test ermöglichte die Erkennung von verborgenen mit Mikrozirkulationsstörungen zusammenhängenden Problemen im Organismus der untersuchten Personen.

Als Elektroden wird in bekannten Geräten [1, 2] Messing eingesetzt. Messing lässt sich beim Kontakt mit der Haut der zu untersuchenden Person nicht polen. Andererseits oxydiert es sehr schnell und intensiv im Laufe der Untersuchung durch die Wirkung von aggressiven biologischen Ausscheidungen der zu untersuchenden Person, insbesondere wenn Stoffwechselerkrankungen vorliegen. Dies ermöglicht es nicht, zuverlässige und reproduzierbare Messergebnisse zu bekommen.

Der Anmelder benutzte zur Fertigung der Elektroden unoxydierbare Metalle, nämlich Titan oder medizinischen Edelstahl. Dabei wurden die Arm- und Beinelektroden mit technischen Einschnitten versehen, um die biologischen Ausscheidungen aus dem Messraum zum Zeitpunkt des Stromlaufs im Stromkreis zu entfernen. Gerade diese Lösung macht die Messungen reproduzierbar.

Die Stirnelektroden (siehe Fig. 6) wurden vom Anmelder auf einem elastischen Reifen befestigt. Der Reifen wurde aus Kunststoff gefertigt. Das ermöglicht es, den Reifen hygienisch problemlos nach jeder Behandlung zu reinigen. Die Reinigung erfolgt mit einem Gazebausch, der mit 70 %-Spiritus angefeuchtet ist.

Die bewegliche Klammer stellt einen optimalen Reifendurchmesser für jede zu untersuchende Person individuell ein. Bei der Standardarretierung der beweglichen Klammer wird das Phänomen der automatischen Anpresskraftregelung der Elektroden an die Stirnhaut der zu untersuchenden Person beobachtet. Das stellt die Reproduzierbarkeit der abgelesenen Kennwerte sicher.

Die Stirnelektroden sind auf einem elastischen Reifen mit einem strikt vorgegebenen für alle geltenden Untersuchungsabstand von 72,3 mm befestigt. Das ist das Knowhow des Verfahrens, weil dieser Abstand experimentell ausgewählt worden ist. Er gilt auch für Kinder ab 3 - 4 Jahre. Das stellt eine Kennziffernstabilität in den ermittelten Datenbanken sowie eine Zuverlässigkeit des vorgeschlagenen Modus der graphischen Auswertung zwecks dynamischer Prüfung und Beurteilung der Homöostasestörungen sicher.

Die Stirnelektroden (Fig. 6) sind als Rundelektroden mit gründlich geschliffener Oberfläche und dem experimentell ausgewählten Durchmesser von 23 mm gefertigt. Der kleinere Durchmesser bedingt Probleme bei Messungen auf einer Trockenhaut. Bei manchen Patienten entstehen Schmerzempfindungen infolge der mechanischen Hautkompression zur Zeit des Stromimpulsverlaufs. Der größere Durchmesser verursacht Probleme bei der Befestigung der Elektroden auf der Stirn, besonders bei Kindern.

Die verstellbare Reifenausführung sowie die Anordnung der Klammer an der Bedienungsseite stellt die bequeme Arretierung der Einrichtung auf dem Kopf der zu untersuchenden Person sicher und schließt eine mechanische Verwechslung der rechten und der linken Elektrode aus.

Die Armelektroden für Segmentdiagnose (siehe [2]) sind zylinderförmig gefertigt und stellen keinen Geräteschutz vor dem Elektrodenschutz zur Zeit der Messungen bzw. der Strombeanspruchungen besonders bei Kindern sicher. Es sind auch Artefakte bei der Ergebnisauswertung möglich. Es liegt keine Normung in Bezug auf Zylinderpresskraft vor. Dabei haben die eigenen Untersuchungen gezeigt, dass ausgerechnet diese Kraft die ermittelten Rohdaten festlegt.

Die Armelektroden (Fig. 7) gemäß der angemeldeten Erfindung werden in Form von Platten gefertigt und auf einem Holzuntersetzer sicher befestigt. Die Ausführung des Holzuntersetzers schließt eventuelle Elektrodenschlüsse, ihre Abtrennung bzw. Verpolung (rechte - linke Elektrode) aus.

Die Beinelektroden werden bei den bekannten Einrichtungen vernachlässigt und als nebensächlich betrachtet.

Gemäß dem angemeldeten Gerätetechnik- und Programmsystem werden die Beinelektroden (Fig. 8) genau wie die Armelektroden aus biologisch inaktiven Stoffen nämlich aus Titan oder medizinischem Edelstahl gefertigt. Dabei werden sie auch mit technischen Einschnitten versehen, um die biologischen Ausscheidungen aus dem Messraum zum Zeitpunkt des Stromverlaufs im Stromkreis zu entfernen. Ähnlich wie bei den Armelektroden, werden die Beinelektroden am Holzuntersetzer befestigt.

Das schließt einen Schluss untereinander sowie die zufällige Verpolung (rechte - linke Elektrode) aus. Es wird eine leicht auseinandernehmbare Bauweise angewendet, um eine prompte hygienische Behandlung zwischen den Untersuchungen einzelner Patienten zu ermöglichen.

Das Wichtigste besteht jedoch darin, dass die Beinelektroden am Holzuntersetzer in einem Abstand befestigt werden. Innerhalb dieses Raums wird eine medizinische Wärmflasche angeordnet. Das stellt behagliche Untersuchungsbedingungen sogar beim Betrieb im Winter sicher. Es werden auch Gefäß- und Muskelnkrämpfe vermieden, welche Artefakte bei der Auswertung der Untersuchungsergebnisse bedingen. Dabei wurde eine erhöhende Heilwirkung der Warmelektroden auf die Homöostasekennwerte der zu untersuchenden Person festgestellt.

Um die Befestigung der zusätzlichen Elektroden zu vereinfachen, werden sie in Form von einem Sauggreifer oder flexibel aus leitfähigem Gummi (Fig. 9) gefertigt und auf der Haut der zu untersuchenden Person am Kragen und Bauchgürtel oder mit Hilfe von externen elastischen Geflechten befestigt.

Die Elektroden und die Kabel des angemeldeten Geräts sind mit Markierungen versehen (rechts - links - Kopf - Arme - Beine, selektiv, oben, unten) und untereinander mittels genormten Anschlussteilen verbunden. Das vereinfacht ihre Instandsetzung und den schnellen Wechsel aus einem Ersatzteilsatz bei eventuellen Defekten der Verbindungskabel. Es wird empfohlen, vor der Untersuchung die Anschlussrichtigkeit und die Polarität der benutzten Elektroden mittels einer Originaltestsoftware zu überprüfen.

Die Messeinheit 26 wird über einen USB-Anschluss mit elektronischer galvanischer Trennung gespeist. Dieser Anschluss wird auch zur Analyse der ermittelten PC-Daten benutzt. Das ist ein zusätzliches Kriterium der elektrischen Sicherheit für die zu untersuchenden Person und des Bedieners.

Die durchgeführten Untersuchungen haben überzeugend bewiesen, dass die ermittelten Kennwerte bei der Messung des Stromzeitverhalten zwischen den angelegten Elektroden zweimal bei direkter und Rückschaltung unter Einsatz von Gleichspannung von 1,2 V von der Reihenfolge des Elektrodenanschlusses und von ihrer Ausgangspolarität abhängen. D. h., bei der Untersuchung der gleichen Person können unterschiedliche Kennwerte erfasst werden, wenn die Messungen von Vorwärts- und Sperrwiderstand mehrfach und in unterschiedlicher Reihenfolge durchgeführt werden.

Beim Betrieb des Geräts wurde auch das Phänomen der Heilwirkung vom eigentlichen Diagnosezyklus festgestellt. Dabei erwiesen sich die dynamischen Verläufe von vielfachen Messungen stabiler, sie waren reproduzierbar und vergleichbar. Und zwar, nun fallen immer mehr Kennwerte unter den Normbereich, das Delta zwischen den Vor- und Rückwärtsmessungen nimmt ab, das Zeigerabschlag-Phänomen des Messgeräts verschwindet.

Der Anmelder hat den Ablauf für den Elektrodenanschluss bei Messungen von oben nach unten und bei der Heilkorrektur von unten nach oben synchron mit der Funktion des Farb- und Schallsynthetisators vorgeschlagen.

Die stabilisierende Heil-Boosterschaltung während der laufenden Untersuchung ist in Fig. 15 abgebildet.

Nach der mathematischen Datenverarbeitung ermittelt die Software die Zonen mit vorliegender Polarisation, d. h., die Zonen mit vorherrschendem «+» und «-» sowie mit Homotoxikose, Anpassungsreserven, Polarisationskennwerte rechts und links, im oberen und im unteren Körperteil, Fallverlauf und Deviation und das Vorhandensein von Kreuzungen (Fig. 17 - 21, 27 - 32). Ausgerechnet dieser Modus ermöglicht es dem Gerätetechnik- und Programmsystem, die individuelle Homöostasekorrektur selektiv vorzunehmen, indem die Strommessimpulse von 1,2 V mit Gegenpolarität an das gewählte Elektrodenpaar (drei, vier, fünf, acht oder neun) gegeben werden. Es wird auch ermöglicht, den Modus für den optimalen galvanischen Ausgleich aufeinander folgend zu wählen (Fig. 24 - 26).

Die Funktionsweise der Einrichtung ist wie folgt:

Der PC wird mit der vorinstallierten Software "IMAGO Diagnose und Therapie" und dem Testprogramm zur Überprüfung des richtigen Anschlusses und Polarität der sechs oder mehr benutzten Elektroden gestartet. Der USB-Anschluss des PC wird an das angemeldete Gerätetechnik- und Programmsystem angeschlossen. Die Anschlüsse werden mit Kabeln und mit Elektroden verbunden. Die Elektroden werden dabei mit einer medizinischen Wärmflasche vorgewärmt. Die Testperson wird vor dem PC-Monitor hingesetzt. Es werden mögliche und zu prognostizierende Homöostasestörungen im Organismus simuliert. Die Simulation erfolgt durch die aufeinander folgende Eingabe der Beschwerden und sonstiger zuverlässiger Daten, welche die vorliegenden Gesundheitsprobleme kennzeichnen, in die Datenbank der SW. Danach werden aufeinander folgend alle Hauptelektroden an der Testperson befestigt: Stirnelektroden mit dosiertem Anpressen an die Haut, Ohrklipps des Kardiotachometers, Armelektroden auf dem Holzuntersetzer, Beinelektroden auch auf dem dielektrischen Untersetzer und mit Vorwärmung mittels medizinischer Wärmflasche, eine (linker Arm) oder mehr (rechter Arm, linker und rechter Oberschenkel) Druckmanschette des Tonometers.

Bei detaillierter Diagnose wird die obere zusätzliche als Sauggreifer oder aus leitfähigem Gummi gefertigte Elektrode Nr. 8 für die Männer auf die hintere Halsoberfläche und die untere Elektrode Nr. 9 in der Nabelprojektion gesetzt. Für Frauen wird die obere Elektrode im Brustbeinbereich und die untere Elektrode in der Kreuzgegend gesetzt. Werden in der Anamnese der zu untersuchenden Person Traumata, Operationen, starke Schmerzen erwähnt, so wird in die betroffene Zone die selektive Elektrode Nr. 7 mit Sauggreifer-Arretierung gesetzt.

Sobald das Gerät eingeschaltet wird, wird an die Hauptelektroden automatisch die Ausgleichsanpassungsboosterspannung von 0,2 V mit vorgegebener Polarität angeschlossen, um die Schadwirkung der Messimpulse zu vermeiden. Vor den Messungen wird das von den Armelektroden und den Ohrklipps kommende kardiologische Ausgangssignal aufgenommen. Während der Untersuchung wird dieses Signal in den Pausen zwischen den Stromimpulsen ständig erneuert.

Die Messungen werden mit einer Spannung von 1,2 V von oben nach unten gemäß der strikt vorgegebenen Reihenfolge angefangen, indem der Vorwärtsstrommessimpuls mit einem Impuls mit umgekehrter Polung abgewechselt wird, um die Elektrolyse der zu untersuchenden Gewebe auszuschließen. Nach der Messung der Ausgangsdaten zur Beseitigung des Nebeneffekts infolge der Messimpulse werden die myostimulierende Heilimpulse an die Elektroden mit vorgegebener Polarität gegeben. Diese Impulsgabe erfolgt mit einem Satz von Referenzfrequenzen (siehe Tabelle 1), welche experimentell ermittelt wurden. Das wird von einem Tonsignal und Blinken des Farbhintergrunds auf dem Monitor in Übereinstimmung mit der Tabelle der durch den Urheber vorgegebenen Werte begleitet.

Bei der Diagnose werden die myostimulierenden Impulse während des 2. Zyklus einzeln gesendet. Danach werden die Messungen wiederholt durchgeführt. An die Elektroden werden wieder Heilimpulse mit veränderbarer Frequenz und Impulsschaltverhältnis angelegt. Jedoch weisen sie diesmal die Form von Impulspaketen à 10 bis 20 Spitzen je nach ihrer Häufigkeit pro Zeiteinheit auf. Die Impulse werden von einem Tonsignal und Blinken des Farbhintergrunds auf dem Monitor in Übereinstimmung mit der Tabelle der durch den Urheber vorgegebenen Werte begleitet.

Nach der 1. Heilwirkung werden bei den meisten Testpersonen (87 %) alle Funktionsänderungen ausgeglichen. Es bleiben nur organische Änderungen übrig, die selektiver Korrektur nach mathematischer Behandlung der Parameter +\- Polarisation bedürfen. Nach dem 2. Zyklus der Heilimpulse bleiben nur schwer korrigierbare Homöostasestörungen übrig, die andere Korrekturverfahren und Verlaufsbeobachtungen voraussetzen.

Für sechs Hauptelektroden entwickelte der Anmelder das Pentagramm-Korrekturverfahren. Bei diesem Verfahren wirkt eine Elektrode, z.B. Stirn rechts (-), als gemeinsame Elektrode, und 5 andere Elektroden haben ein (+). Es wurden auch andere Modi der Galvanokorrektur bei unterschiedlichen Kombinationen der Haupt- und der zusätzlichen Elektroden gebildet, die unten angeführt sind (s. Tabelle 5, Fig. 23-26).

Die Homöostase gilt als wiederhergestellt, wenn alle Kennwerte bei allen Ableitungen normalisiert sind, wenn keine Kreuzungen, Fälle, Deviationen vorliegen und die Normspannweite erreicht ist.

Lassen sich die Homöostasekennwerte nicht wiederherstellen, so werden spezifische physiotherapeutische Impulse über den Anschluss des externen Generators selektiv an das vorgegebene Paar bzw. Kombination der Elektroden bis zur vollständigen Wiederherstellung der festgestellten Homöostasestörungen gegeben. Die Impulsgabe erfolgt mittels eines medizinischen Elektrostimulationsgeräts "DiaDENS-T". Alternativ werden die problembehafteten Hautbereiche mit dem Farbsynthetisator "GESKA-POLITSVET" behandelt. Die Informationsaufnahme wird dabei im dynamischen Verlauf wiederholt.

Tabelle 1:

| Galvanische Hauptkorrekturpaare (-) und Referenzfrequenzen der myostimulieren den Heilimpulse und ihre Verknüpfung mit Ton- und Farbtherapiebetrieben. | | | | | | | | | |
|---|---|---|---|---|---|---|---|---|---|
| Ableitungsnummer | 1 | 4 | 5 | 6 | 7 | 10 | 11 | 12 | 15 |
| Note | H-0 | D | D# | E | F | G# | A | A# | C2 # |
| Frequenz | 0,38 5 | 2,31 | 3,08 | 3,85 | 4,62 | 6,93 | 7,7 | 8,47 | 10,78 |
| Impulsverhältnis | 99 | 66 | 57 | 48 | 41 | 22 | 17 | 12 | 3 |
| Farbe (Spektrum) | Dunkel rot | Gelb | Gelb-Grün | Grün | Blau | Hellviolett | ViolEtt | Dunkelviolett | Hellgrau |
| Ableitung/ galvanisches Paar | 3-4 | 2-4 | 3-5 | 6-4 | 3-1 | 2-5 | 6-5 | 2-1 | 1-6 |

Tabelle 2:

| Ausgleichende (+\- zusammengesetzte) galvanische Korrekturpaare und Referenzfrequenzen der myostimulierenden Heilimpulse und ihre Verknüpfung mit Ton- und Farbtherapiebetrieben | | | | | | |
|---|---|---|---|---|---|---|
| Nr der Ableitung | 2-direkte | 2-inverse | 8-direkte | 8-inverse | 13-direkte | 13-inverse |
| Note | C 1. Oktave | C 1. Oktave | F# | F# | H | H |
| Frequenz | 0,77 | 0,77 | 5,39 | 5,39 | 9,24 | 9,24 |
| Impulsverhältnis | 88 | 88 | 34 | 34 | 9 | 9 |
| Farbe | Rot | Rot | Blau | Blau | Dunkelviolett | Dunkelviolett |
| Ableitung | 4-5 | 5-4 | 4-1 | 1-4 | 5-1 | 1-5 |

Tabelle 3:

| Ausgleichende (-\+ zusammengesetzte) galvanische Korrekturpaare und Referenzfrequenzen der myostimulierenden Heilimpulse und ihre Verknüpfung mit Ton- und Farbtherapiebetrieben für benutzte Ableitungen mit 6 Elekroden: | | | | | | |
|---|---|---|---|---|---|---|
| Nr der Ableitung | 14-direkte | 14-inverse | 9-direkte | 9-inverse | 3-direkte | 3-inverse |
| Note | H andere Oktave | H andere Oktave | G | G | C# | C# |
| Frequenz | 10,01 | 10,01 | 6,16 | 6,16 | 1,54 | 1,54 |
| Impulsverhältnis | 6 | 6 | 27 | 27 | 77 | 77 |
| Farbe | Dunkelgrau | Dunkelgrau | Türkisblau | Türkisblau | Orange | Orange |
| Ableitung | 6-2 | 2-6 | 6-3 | 3-6 | 2-3 | 3-2 |

Tabelle 4:

| Inverse galvanische (+) Hauptkorrekturpaare und Referenzfrequenzen der myostimulierenden Heilimpulse und ihre Verknüpfung mit Ton- und Farbtherapiebetrieben für die benutzten Ableitungen mit 6 Elektroden. | | | | | | | | | |
|---|---|---|---|---|---|---|---|---|---|
| Nr. | 1 | 4 | 5 | 6 | 7 | 10 | 11 | 12 | 15 |
| Note | H-0 | D | D# | E | F | G# | A | A# | C2# |
| Frequenz | 0,385 | 2,31 | 3,08 | 3,85 | 4,62 | 6,93 | 7,7 | 8,47 | 10,78 |
| Impulsverhältnis | 99 | 66 | 57 | 48 | 41 | 22 | 17 | 12 | 3 |
| Farbe (Spektrum) | Dunkelrot | Gelb | Gelbgrün | Grün | Blau | Hell violett | Violett | Dunkel violett | Hellgrau |
| Ableitung - galvanisches Paar | 6-1 | 1-2 | 5-6 | 1-3 | 4-6 | 5-2 | 4-2 | 5-3 | 4-3 |

Tabelle 5:

| PENTAGRAMM-Anschlussvarianten von Hauptelektroden bei der Korrektur von Homöostasestörungen. | | | | |
|---|---|---|---|---|
| Variante Nr. | Gemeinsame Elektrode | Polarität der gemeinsamen Elektrode | Pentagramm für galvanische Korrektur | Polarität der Pentagrammelektrode n |
| 1. | Stirn links | (-) | BL-AL-AR-BR-SR | (+) |
| 2. | Stirn links | (+) | SR-BR-AR-AL-BL | (-) |
| 3. | Stirn rechts | (-) | SL-BL-AL-AR-BR | (+) |
| 4. | Stirn rechts | (+) | BR-AR-BL-AL-SL | (-) |
| 5. | Stirn beidseitig | (-) SL SR | BL-AL-BR-AR-SR SL-AL-BL-AR-BR | (+) |
| 6. | Stirn beidseitig | (+) SR SL | BR-AR-AL-BL-SL SR-BR-AR-AL-BL | (-) |
| 7. | Arm rechts | (-) | AL-SR-BL-SL-BR | (+) |
| 8. | Arm rechts | (+) | SL-BR-SR-BL-AL | (-) |
| 9. | Arm links | (-) | SR-AR-BR-SL-BL | (+) |
| 10. | Arm links | (+) | BL-SL-BR-AR-SR | (-) |
| 11. | Bein rechts | (-) | BL-SR-AL-SL-AR | (+) |
| 12. | Bein rechts | (+) | AR-SL-AL-BL-SR | (-) |
| 13. | Bein links | (-) | AL-SR-BR-AR-SL | (+) |
| 14. | Bein links | (+) | SL-BR-AR-SR-AL | (-) |

Die Möglichkeiten von Diagnose und Korrektur der Homöostase durch das angemeldete komplexe Gerätetechnik- und Programmsystem werden durch folgende klinische Beispiele bestätigt:

Beispiel 1: 22.12.2005. Patient Fok... Ju.P., 69 J. Festgestellte Rg-Diagnose: Mittelfelllymphom, Winkelblockglaukom, Erblindung des linken Auges. Der Patient raucht zu viel.

Während der Aufnahme von Leitfähigkeitskenndaten wurden Kreuzungen zwischen der Stirn rechts und dem Arm links festgestellt. Das zeugte von schweren Homöostasestörungen. In den Absorptionsbereichen waren die Kennwerte des linken Auges schlimmer als die des rechten. Die Reaktion auf Beanspruchung kommt hauptsächlich von der linken Schulter. Bei graphischer Darstellung gibt das Auslegeprogramm die Absorptionsbereiche in der Projektion der Thymusdrüse und der retrosternalen Lymphknoten. Es wurde +\- Galvanokorrektur vorgenommen.

05.05.2006 - Die Kreuzungen lassen sich nicht feststellen. Die Absorptionsbereiche bleiben an derselben Stelle. Die Anpassungsreserven nahmen von 28,6 % bis 30,6 % zu.

Die (+) Polarisation nahm von 920 KE (konventionelle Einheiten) bis 297 und am 21.06.2006 bis auf 3 KE ab.

Damit wurde die 100%-ge Diagnose des beim zu untersuchenden Patient vorliegenden Mittelfelllymphoms sowie der Heileffekt von der eigentlichen Diagnose und Stabilisation der Homöostase im dynamischen Verlauf bestätigt.

Beispiel 2: 09.02.2006: Patientin Sakh... M.A. 75 J. Beschwerden über Obstipationen. Abführmittel hatten keinen Effekt. Traditionelle Behandlungsverfahren ergaben keine Wirkung innerhalb von 2 Jahren. Bei eingehender Diagnose unter Einsatz einer zusätzlichen oberen und unteren Elektrode wurden keine Absorptionsbereiche in der Projektion des Dickdarms festgestellt. Die Reaktion kam von den Gehirnblutgefäßen und von Parathyreoidea, die für den Mineralienaustausch (Kalziumaustausch) im Körper zuständig ist. Die erneute Untersuchung am 06.05.2006 bestätigte dasselbe klinische Bild. Die Verordnung von komplexen Mineralstoffpräparaten mit andauernden Behandlungskuren ermöglichte es, das Problem zu lösen.

Beispiel 3: Patient Sor... A.D. 44 J. Reihenuntersuchung. Es lagen keine Beschwerden vor. Die Absorptionsbereiche wurden in der Leber- und Prostataprojektion festgestellt. Endgültige Anamnese: Infektiöse Hepatitis. Der Patient machte eben eine urologische Behandlung im Zusammenhang mit Prostataentzündung durch. Die Absorptionsbereiche auf der Leber können als Erschwerung infolge der Medikamenteneinnahme bei schwacher Leber (toxische Hepatitis) gedeutet werden. Die Absorptionsbereiche an der Vorsteherdrüse können als chronische Auswirkungen der behandelten Prostataentzündung ausgelegt werden. Das bedarf einer Dauerbehandlung dieser Krankheit mit nachfolgenden Reizproben.

Beispiel 4: Patient Eit... A.L. 61 J. Beschwerden über schmerzhaftes Harnlassen. Während der Untersuchung wurden Absorptionsbereiche in der Projektion der Harnblase und beider Nieren festgestellt. Anamnese: Appendektomie. Die 9. selektive Sauggreifer-Elektrode wurde in der Narbenprojektion, nämlich rechts in der Leisten- und Krummdarmgegend befestigt. Es wurde festgestellt, dass die linke Niere intensiver als die rechte reagiert. In der Projektion des Blinddarms wurde Adhäsionsprozess erkannt. Es wurde der externe Generator angeschlossen. Die selektive Einwirkung wurde in Bezug auf die Selektivelektrode in der Kombination mit der rechten und der linken Beinelektrode vorgenommen.

DS: Pyelozystitis. Die verordnete Behandlung ergab eine schnelle Verbesserung des klinischen Bildes.

Beispiel 5: Patientin Art... Ju.I., 24 J. Während der US-Untersuchung wurde Diagnose Thyreotoxikose gestellt. Es lag sichtbarer Exophthalmus (Glotzauge) vor. Die Anzeigeeinheit des Kardiotachometers zeigte Tachykardiekennzeichen an. Die 7. Elektrode aus leitfähigem Gummi wurde aufs Brustbein und die 8. Elektrode im Kreuzgebiet angelegt. Die Absorptionsbereiche wurden in der Projektion der Schilddrüse rechts festgestellt. Die Homöostasekorrektur erfolgte mittels einer Aktivierung der Strombeanspruchung (+) für die (-) Polarisation. Dadurch stiegen die Anpassungsreserve von 28,6 % bis auf 30,3 % an und die Absorptionsbereiche verlagerten sich im dynamischen Verlauf nach unten. Damit wurde die so genannte Hering-Regel repräsentiert. Diese Regel ist für das Informationskorrekturverfahren typisch: Die Krankheit geht von oben nach unten und von innen nach draußen. Einen Monat später erfolgte die dynamische Beobachtung. Der Exophthalmus verringerte sich. Die Kennwerte der Anpassungsreserven blieben bei 30,1 %.

Somit kann diese Erfindung im Rahmen von jährlichen Massenreihenuntersuchungen in Großbetrieben mit gesundheitsschädlichen Produktionsabläufen eingesetzt werden, um die ersten Homöostasestörungen rechtzeitig zu erkennen, sie zu korrigieren und ihren Verlauf zu beobachten.

**Patentansprüche**

1. Komplexes Gerätetechnik und Programmsystem zur Diagnose und Korrektur von Homöostasestörungen mit einem Stromimpulsgeber (22) mit einer Schalteinheit (23), einer Steuereinheit (24) mit einem Kabel zum Anschließen von drei Paaren von Hauptelektroden (25) nämlich Stirn- (1, 2), Arm- (3, 4) und Beinelektroden (5, 6), einer Messeinheit (26) mit einem A/D-Umsetzer und Tonbegleitung und mit einem PC (27) mit Software (SW) zur Ausführung von drei Mess- und zwei Strombelastungsbeispielen in einer vorgegebenen Reihenfolge und graphischen Darstellungen,
**dadurch gekennzeichnet,**
**dass** die Klangfarbe der Tonbegleitung der Messeinheit (26) mit dem Alter und dem Geschlecht der zu untersuchenden Person verknüpft ist,
**dass** an die Schalteinheit (23) mindestens ein Schallsynthetisator (28) mit Farbsynthetisator, eine Steuereinheit (29) zur Steuerung von Druckmanschetten eines Standardtonometers und drei zusätzliche Elektroden (7, 8, 9) angeschlossen sind,
**dass** eine dieser (selektive) Elektroden (7) auf dem Körper der zu untersuchenden Person im Problembereich (Narbe, Schmerzen u.a.m.) befestigt ist,
**dass** die zweite (obere) Elektrode (8) in der Ebene der ersten Brustwirbel befestigt ist,
**dass** die dritte (untere) Elektrode (9) in der Nabelhöhle befestigt ist,
**dass** alle Hauptelektroden (25) mit einer Booster-Korrektureinheit (34) mit einer Spannung von 0,2 V ausgerüstet sind,
**dass** ein Kardiotachometer (31) an die Messeinheit (26) angeschlossen ist, der mit einer Anzeige und einem Speicher ausgerüstet ist und
**dass** die Software (SW) zusätzlich klinische und topographisch-anatomische Nachbildung der vermutlichen Homöostasestörungen bei der Testperson sicherstellt, wobei die visuelle Darstellung dieser Homöostasestörungen in Form von graphischen Abbildungen der Stromimpulsverläufe an anatomischen Computermodellen der Hauptorgane des menschlichen Körpers erfolgt, wobei der Stromimpulsgeber (22) anstatt der Beanspruchungsimpulse die regenerierenden myostimulierenden Impulse zweier Arten, nämlich die Einzel- und die zyklischen Impulse mit veränderbarer Frequenz, Impulsverhältnis und Amplitude, bildet und wobei diese mit den Tonsignalen des Schallsynthetisators (28) mit dem Farbhintergrund des Farbsynthetisators synchronisiert sind.

2. Komplexes Gerätetechnik- und Programmsystem nach Anspruch 1,
**dadurch gekennzeichnet,**
**dass** die Booster-Korrekturereinheit (34) mit einer Gleichspannung von 0,2 V an die Hauptelektroden (25) individuell in vorgegebener Reihenfolge angeschlossen ist und
**dass** alle Eigenstromquellen untereinander galvanisch getrennt sind, einen hohen Ausgangswiderstand aufweisen, sich vor dem Diagnoseanfang einschalten und eine vorgegebene Polarität haben.

3. Komplexes Gerätetechnik- und Programmsystem nach Anspruch 1,

**dadurch gekennzeichnet,**
**dass** alle Elektroden (7, 8, 9) aus einem unmagnetischen und unoxydierbaren Metall gefertigt sind,
**dass** die Stirnelektroden (7) auf einem flexiblen Reifen angeordnet sind, der das regelbare Anpressen an die Stirnhaut während der Diagnose sicherstellt,
**dass** die Arm- (8) und die Beinelektroden (9) mit Einschnitten zum Schweißabfluss aus dem Elektrodenraum versehen und an individuellen Untersätzen arretiert sind,
**dass** die zusätzliche selektive Elektrode (7) in Form von einem Sauggreifer ausgebildet ist und
**dass** die anderen zusätzlichen Elektroden (8, 9) aus flexiblem, leitfähigem Gummi gefertigt und mit einem Thermostabilisator versehen sind.

4. Komplexes Gerätetechnik- und Programmsystem nach Anspruch 1,
**dadurch gekennzeichnet,**
**dass** die Klangfarbe der Tonbegleitung der Messeinheit (26) gemäß einer Formel je nach Alter und Geschlecht der zu untersuchenden Person berechnet ist zu:

$$F = 440 - W * k \text{ (Hz)}$$

wobei sind F die Tongeneratorfrequenz, W das Alter der zu untersuchenden Person, k ein Vervielfachungsfaktor ist, der für Männer 2,741 und für Frauen 2,120 beträgt.

Fig. 1

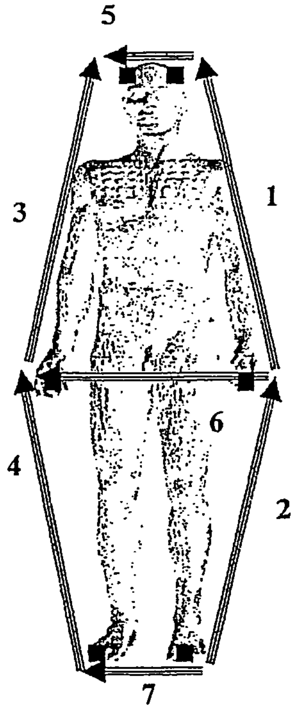


Fig. 2

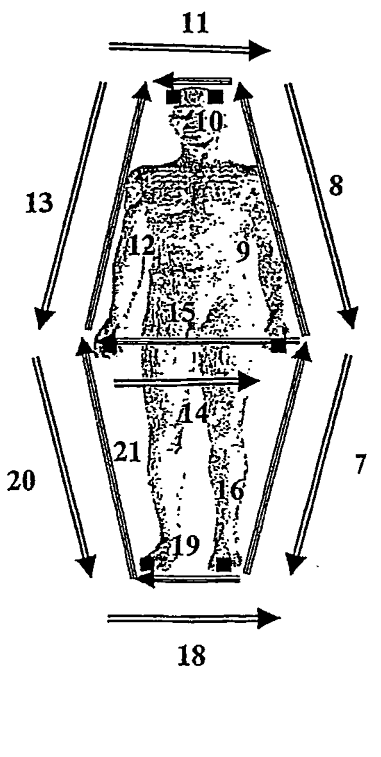

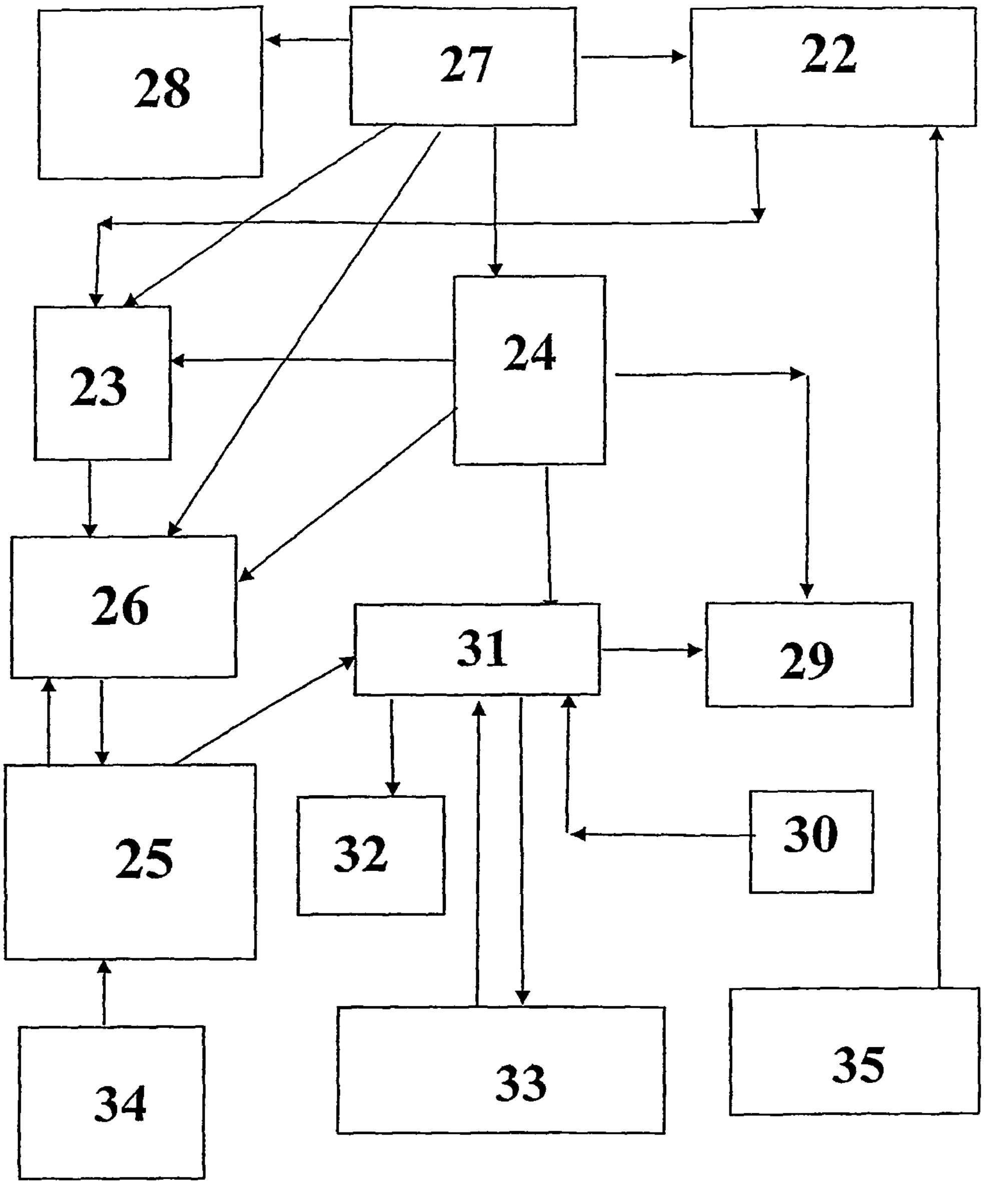


Fig. 3

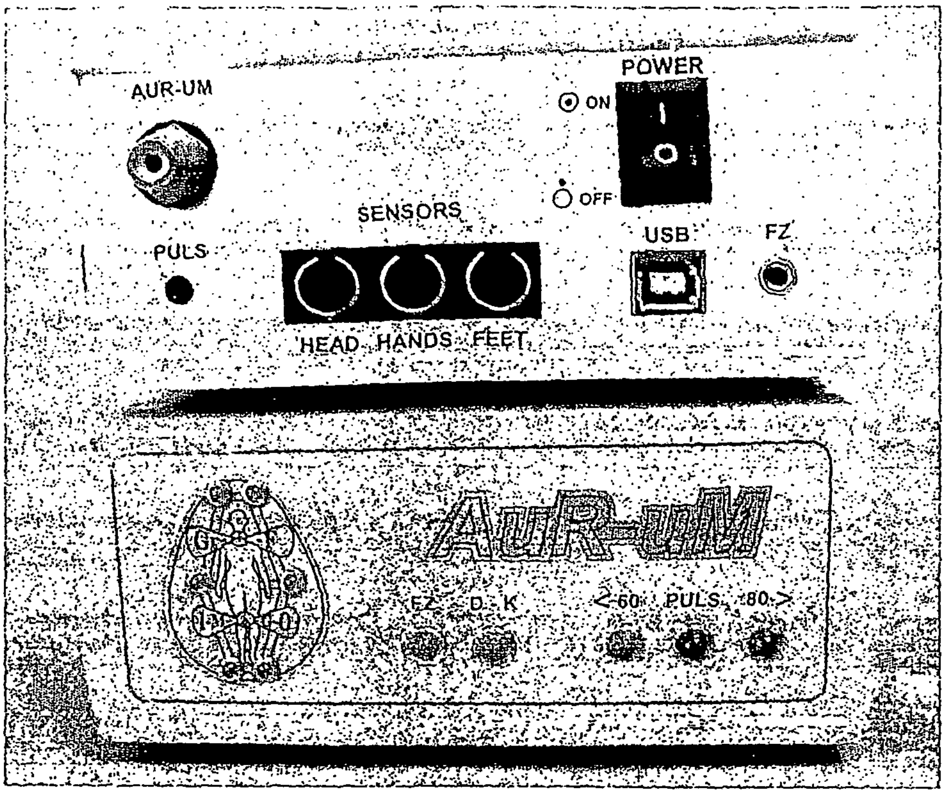
AUR-UM
POWER
ON
OFF
SENSORS
PULS
HEAD HANDS FEET
USB
FZ
AuR-uM
FZ D K
<60 PULS 80>

Fig. 4

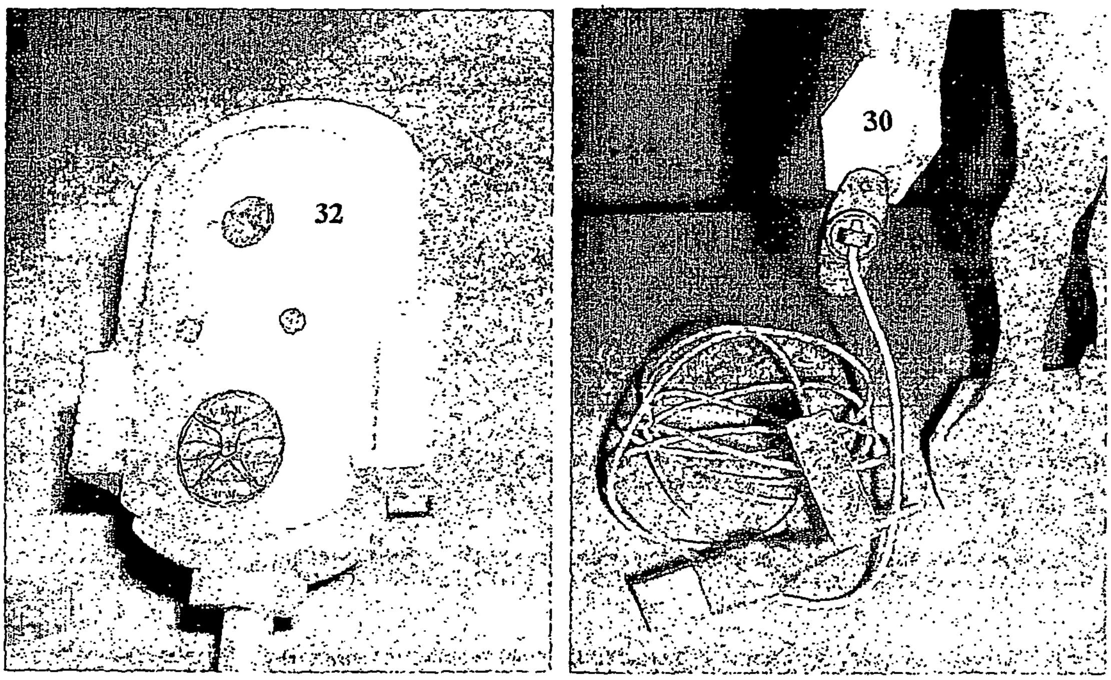
32
30

Fig. 5

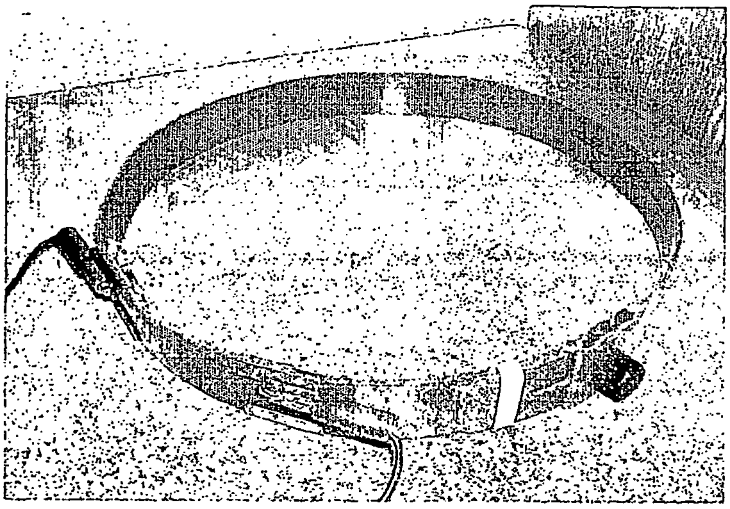

Fig. 6

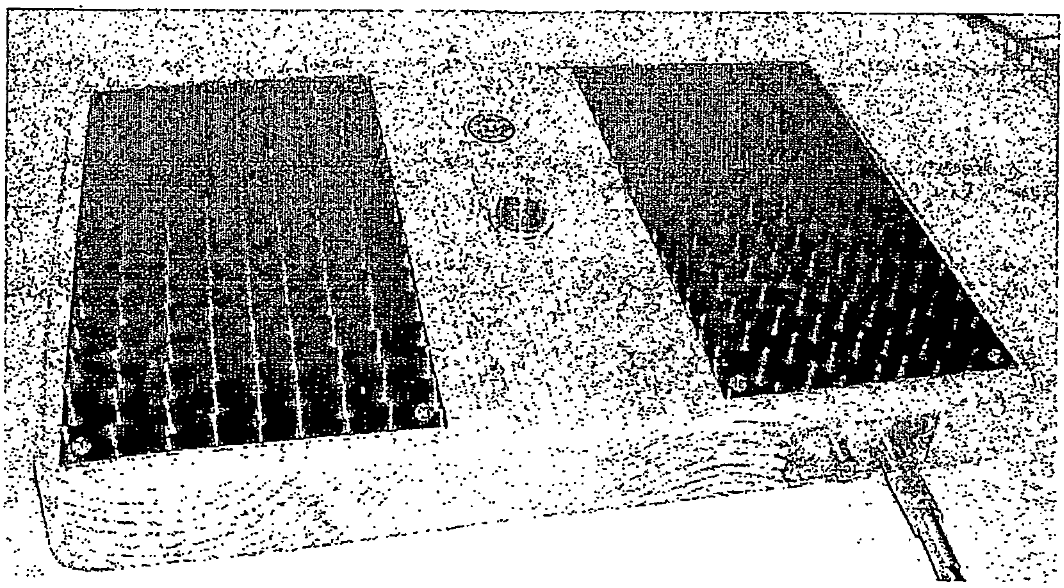

Fig. 7

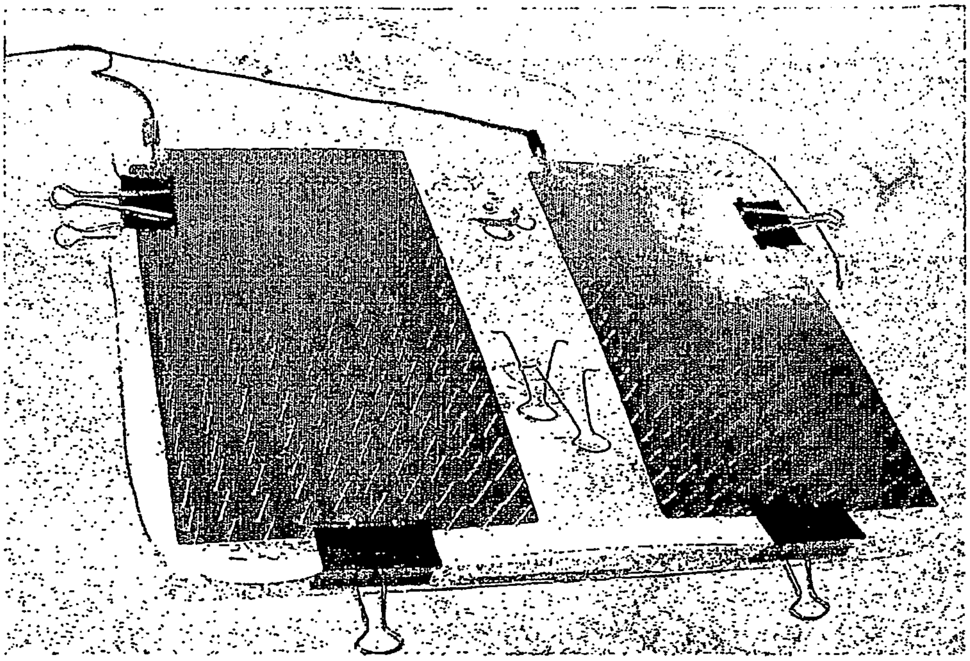

Fig. 8

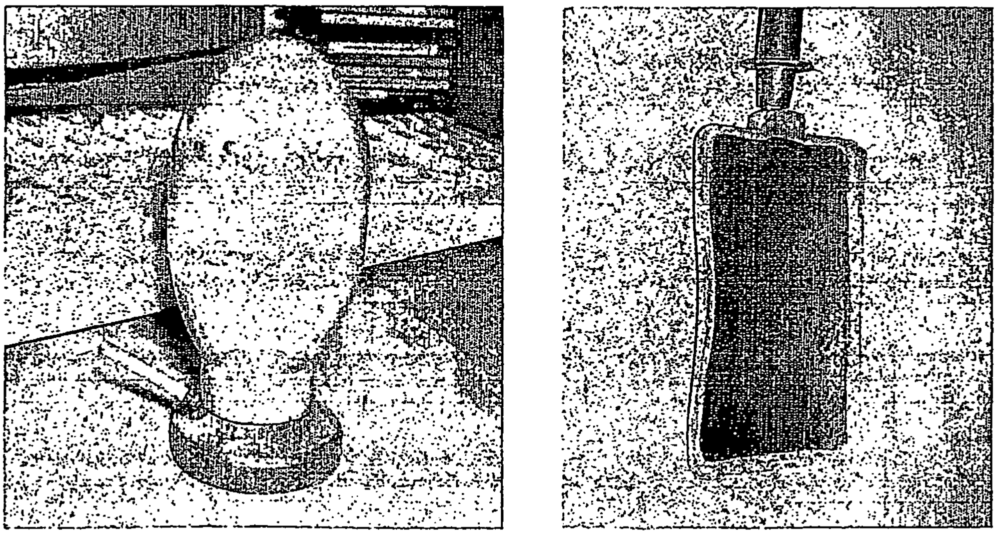

Fig. 9

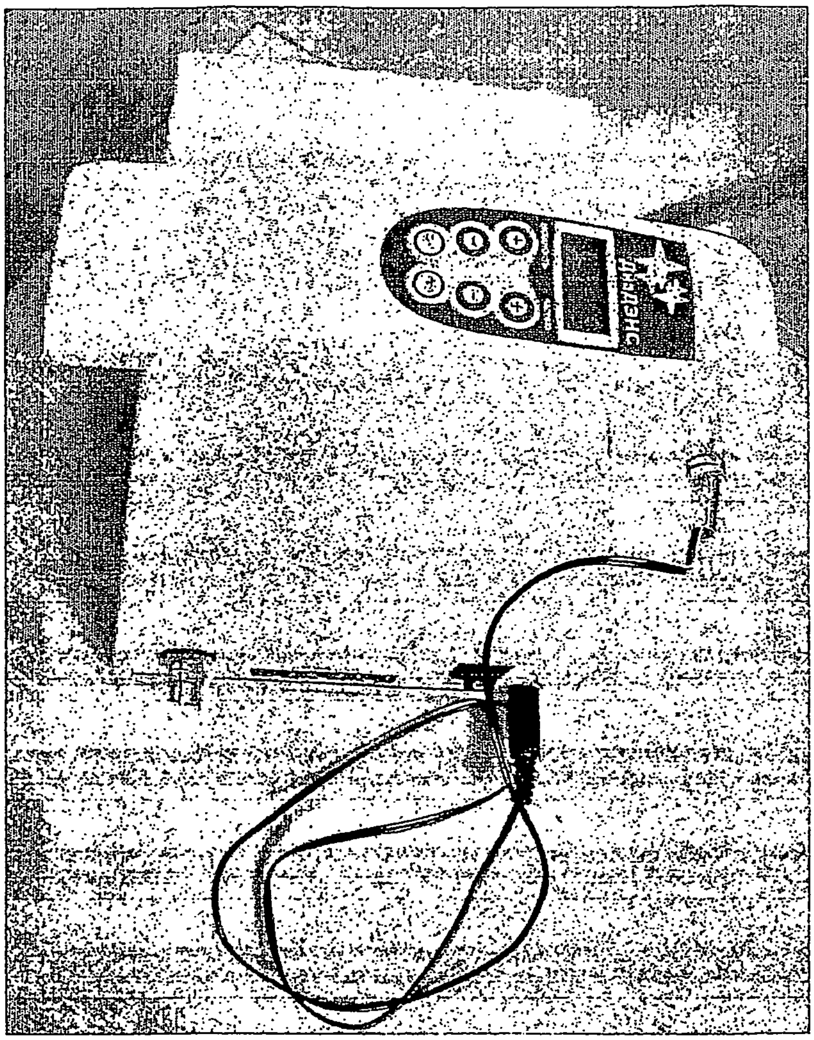

Fig. 10

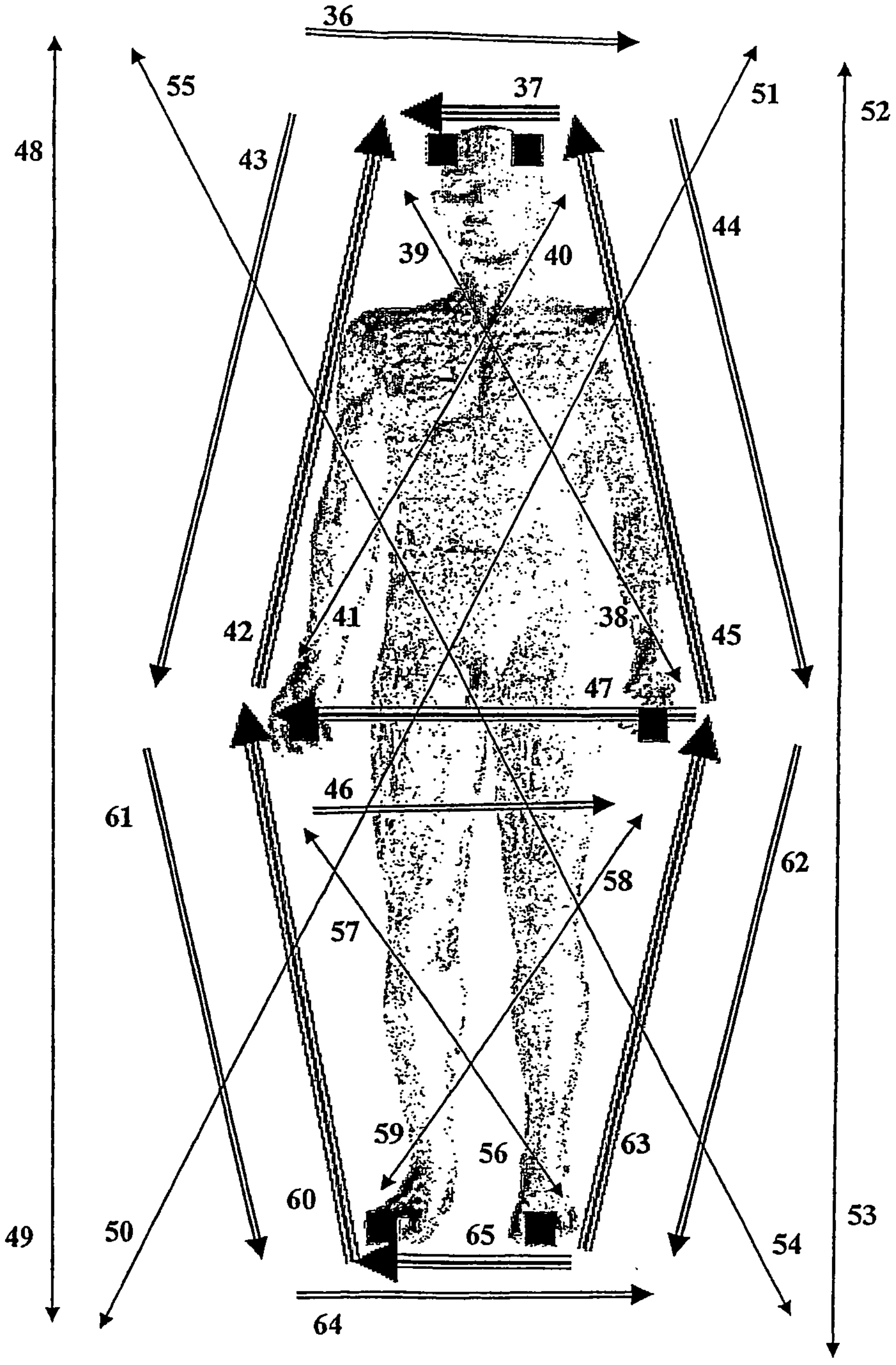
36
37
48
55
43
51
52
44
39
40
42
41
38
45
47
46
61
62
57
58
59
56
63
60
49
50
65
54
53
64

Fig. 11

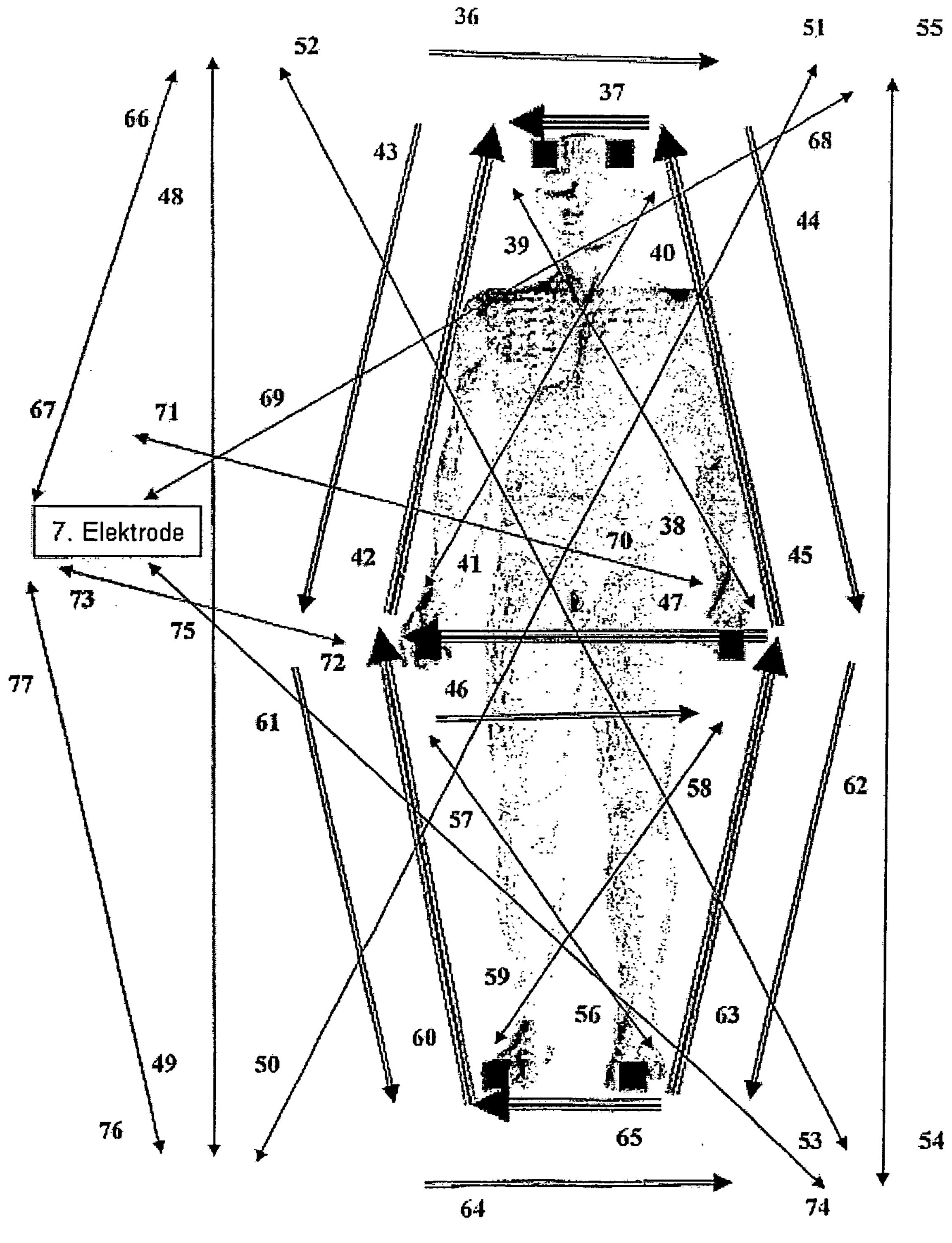
36
52
51
55
37
66
43
68
48
44
39
40
67
71
69
7. Elektrode
38
70
42
41
45
73
47
75
72
77
46
61
58
62
57
59
56
63
60
49
50
76
65
53
54
64
74

Fig. 12

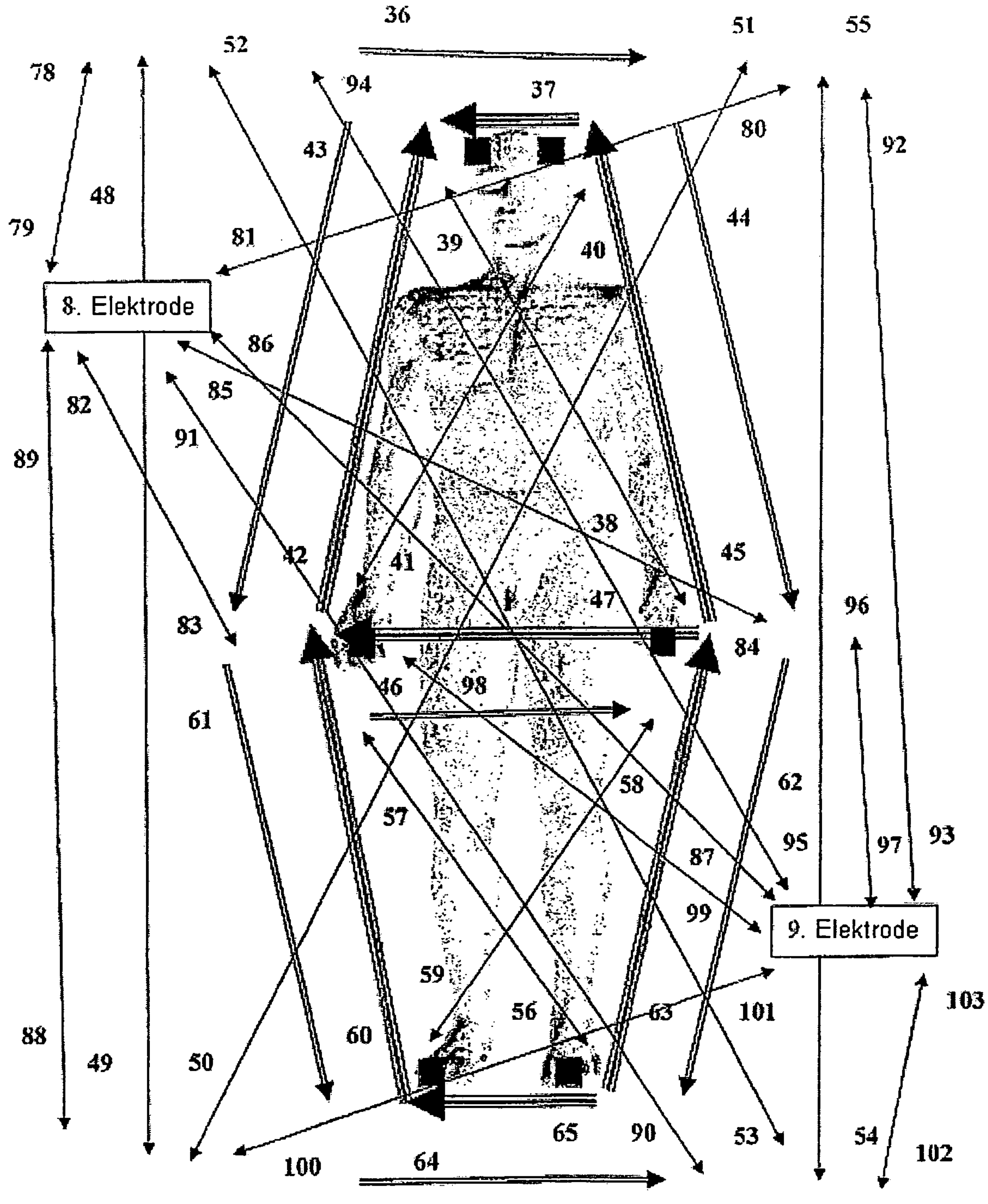


Fig. 13

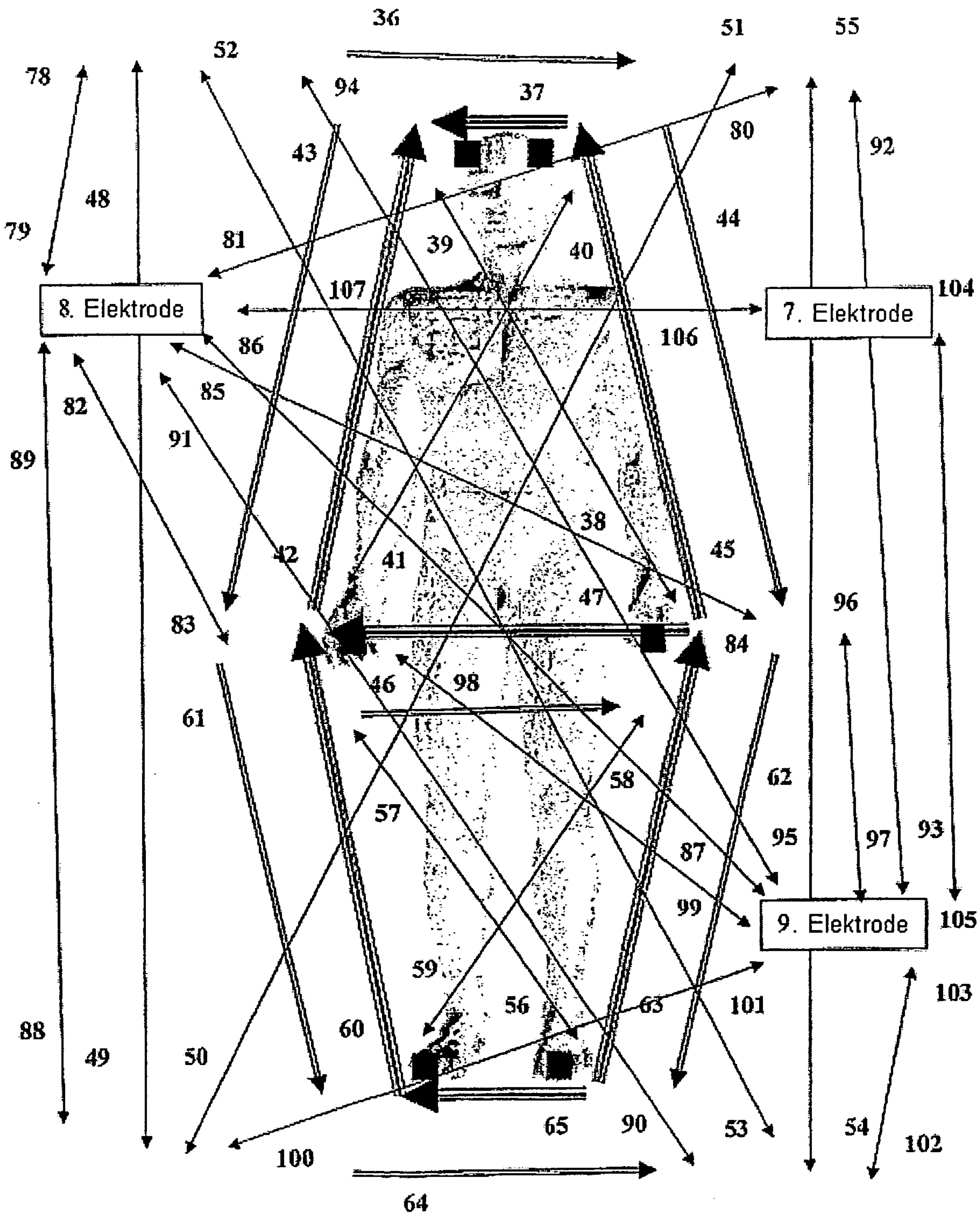
8. Elektrode
7. Elektrode
9. Elektrode
36
37
38
39
40
41
42
43
44
45
46
47
48
49
50
51
52
53
54
55
56
57
58
59
60
61
62
63
64
65
78
79
80
81
82
83
84
85
86
87
88
89
90
91
92
93
94
95
96
97
98
99
100
101
102
103
104
105
106
107

Fig. 14

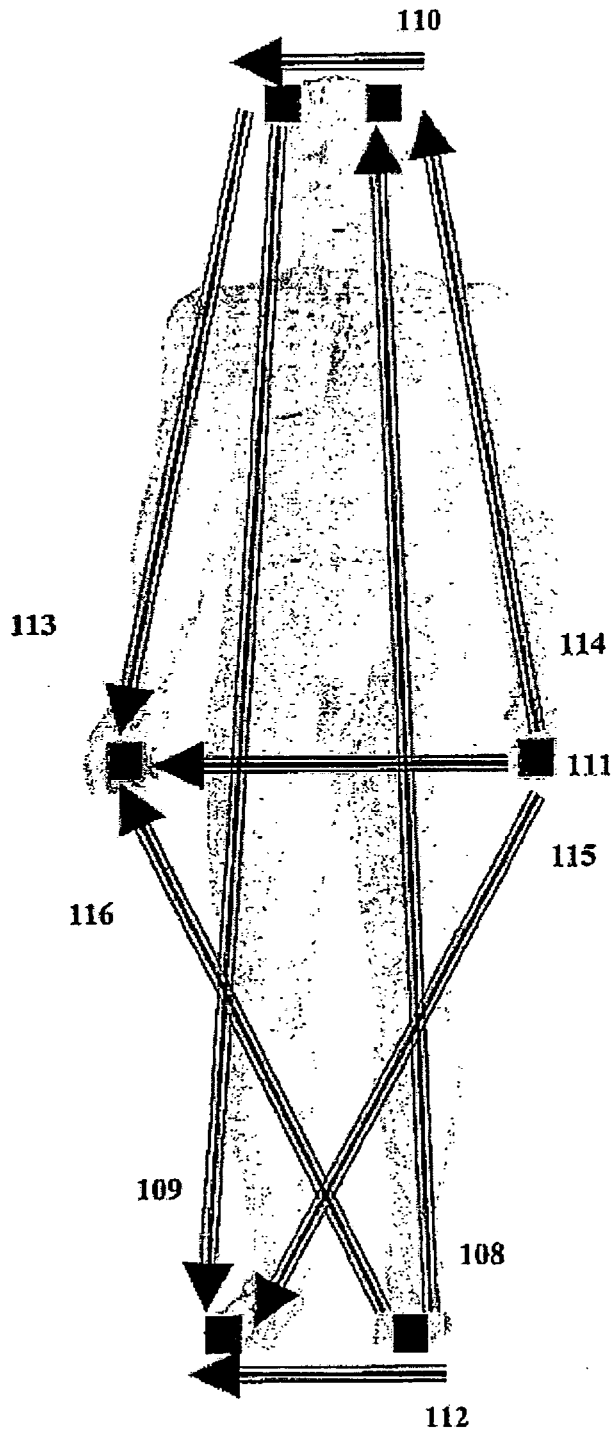


Fig. 15

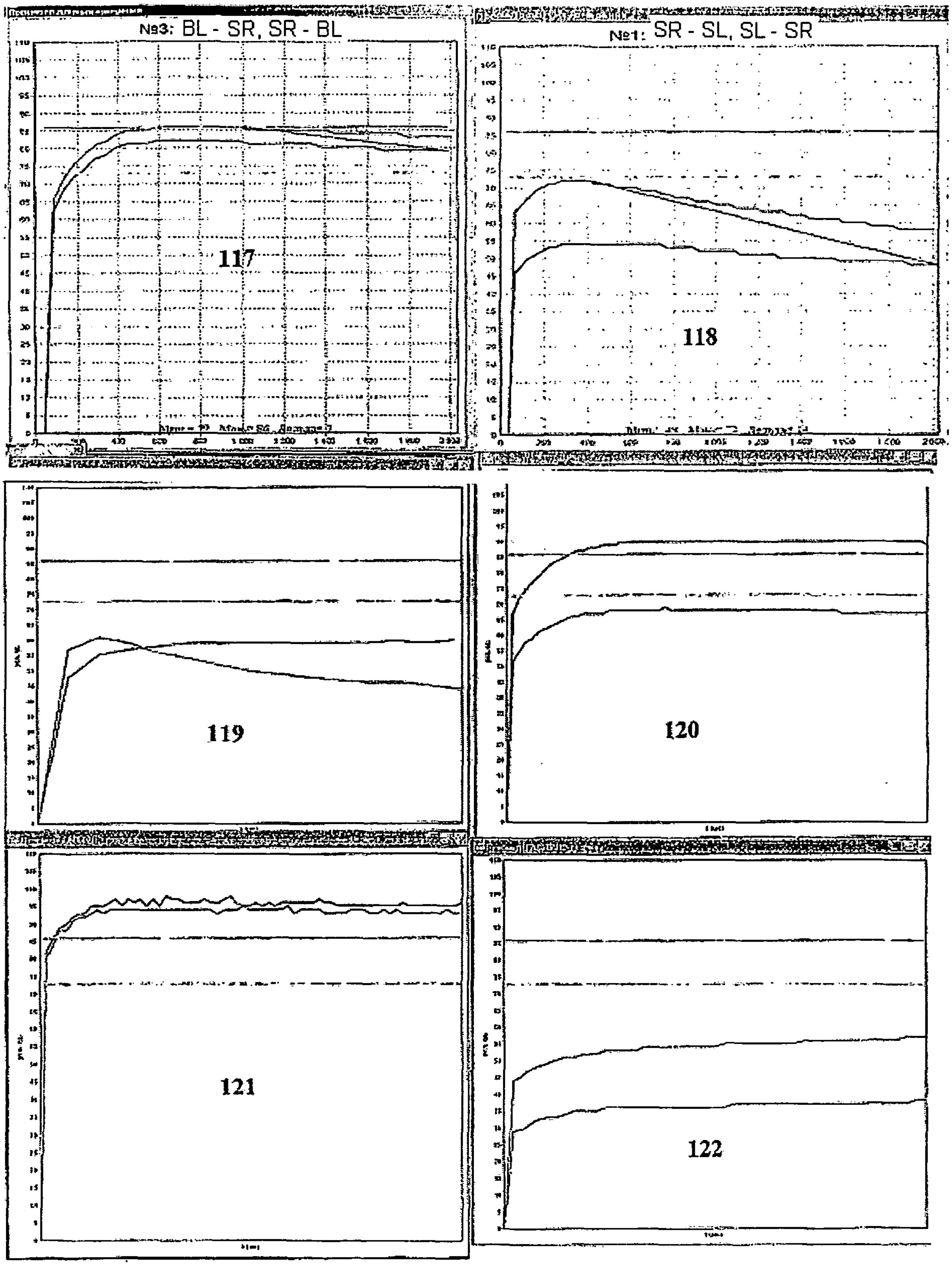


Fig. 16

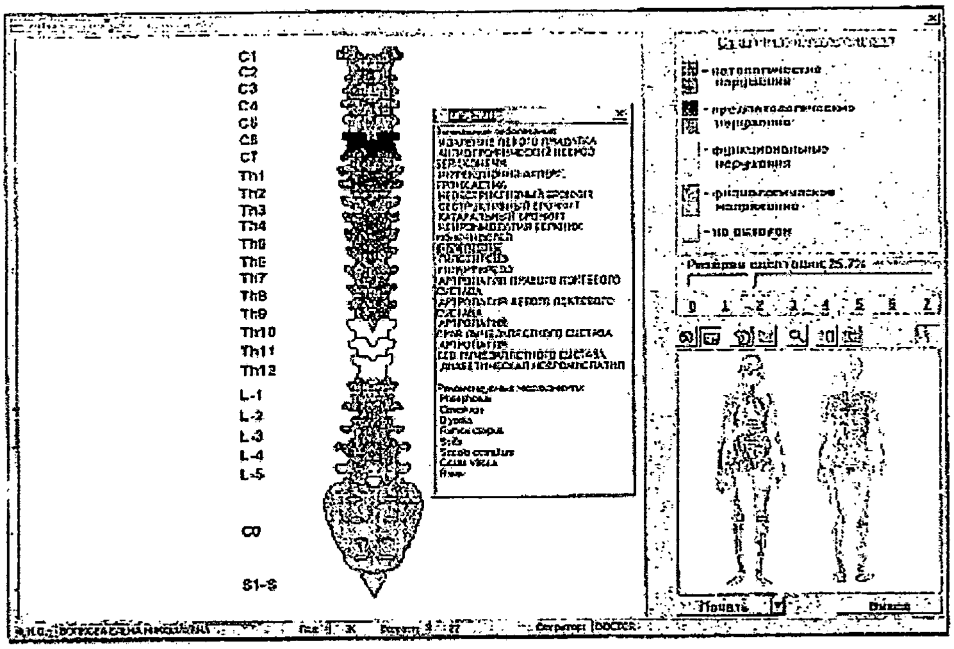
C1
C2
C3
C4
C5
C6
C7
Th1
Th2
Th3
Th4
Th5
Th6
Th7
Th8
Th9
Th10
Th11
Th12
L-1
L-2
L-3
L-4
L-5
S1-S

Fig. 17

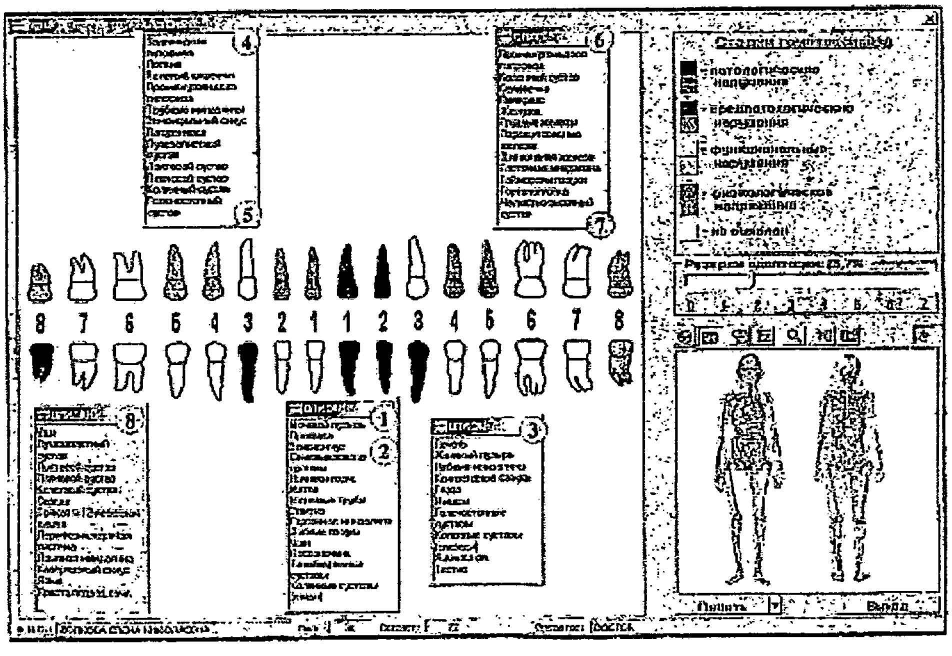

Fig. 18

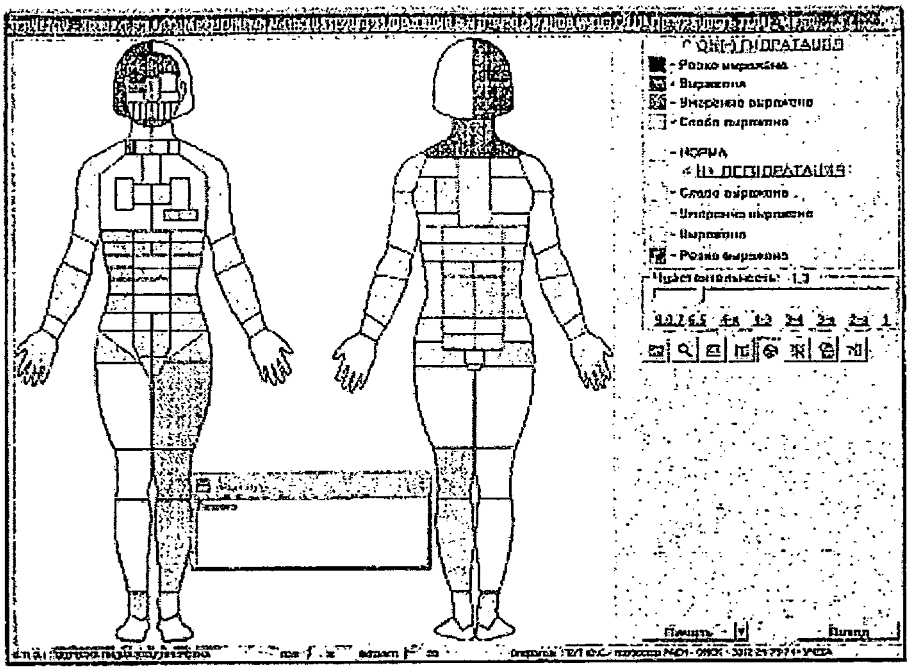

Fig. 19

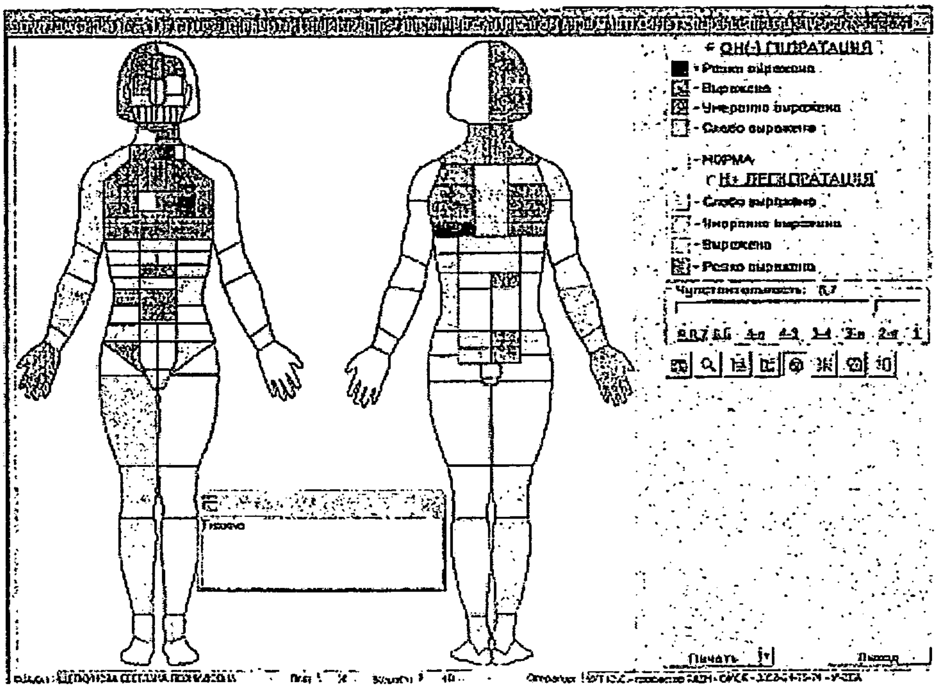

Fig. 20

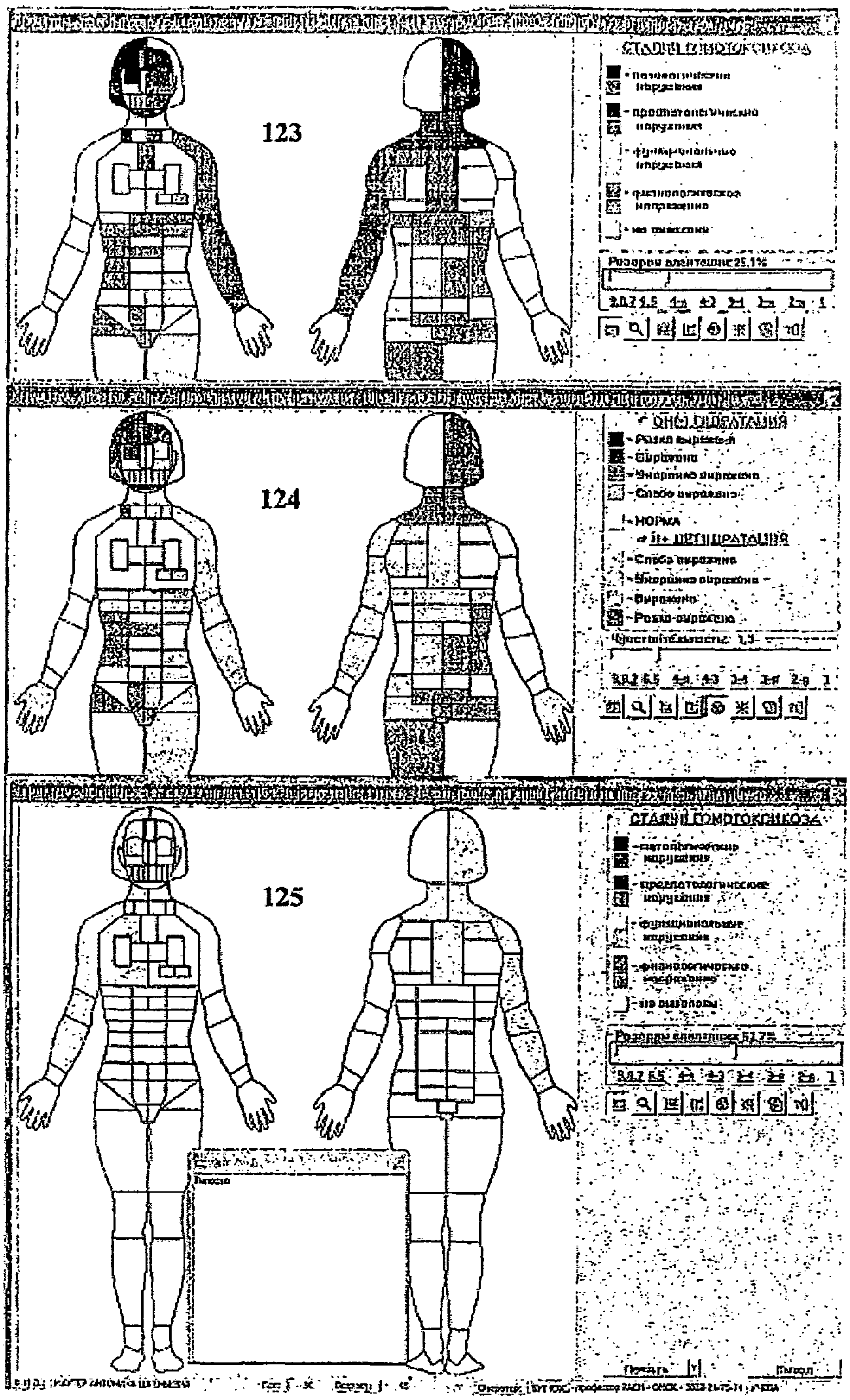


Fig. 21

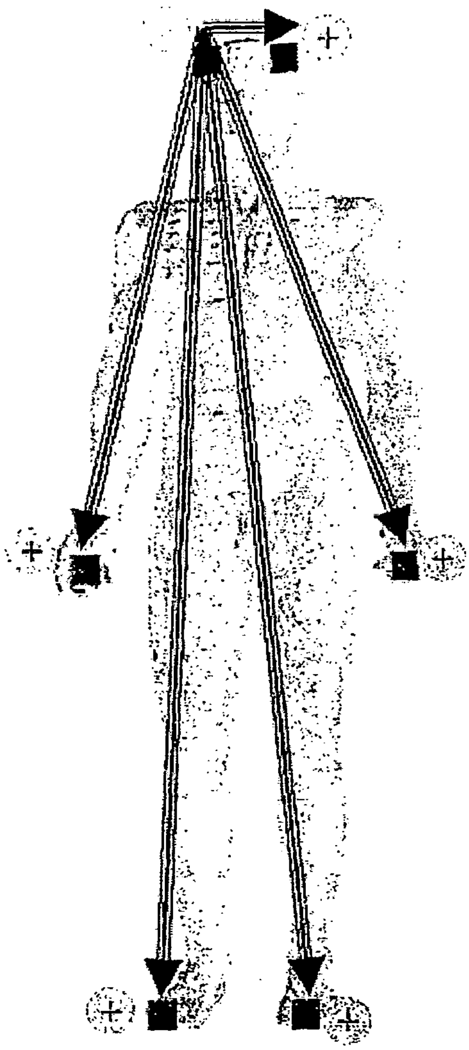

Fig. 22

LISTE DER DURCHGEFÜHRTEN GALVANISCHEN UNTERSUCHUNGEN

01 - HEMISPHÄRE RECHTS (+5) – LINKS H(-5)
02 - HEMISPHÄRE LINKS (+5) – LINKS H(-5)
03 - STIRN LINKS (-) – ALLE ELEKTRODEN(+) - STROM-REPRINTER
04 - STIRN LINKS (+) – ALLE ELEKTRODEN(-) - STROM-REPRINTER
05 - STIRN RECHTS(-) – ALLE ELEKTRODEN(+) - STROM-REPRINTER
06 - STIRN RECHTS(+) – ALLE ELEKTRODEN(-) - STROM-REPRINTER
07 - STIRN BEIDSEITIG(-) – ALLE (+) - STROM-REPRINTER
08 - STIRN BEIDSEITIG (+) – ALLE (-) - STROM-REPRINTER
09 - STIRN BEIDSEITIG L(-) – R(+) - STROM-REPRINTER
10 - STIRN BEIDSEITIG L((+) – R(-) - STROM-REPRINTER
11 - OBERER KÖRPERTEIL (+) - STROM-REPRINTER
12 - OBERER KÖRPERTEIL (-) - STROM-REPRINTER
13 - ARM RECHTS(-) – ALLE ELEKTRODEN(+) - STROM-REPRINTER
14 - ARM RECHTS(+) – ALLE ELEKTRODEN(-) - STROM-REPRINTER
15 - ARM LINKS(-) – ALLE ELEKTRODEN(+) - STROM-REPRINTER
16 - ARM LINKS(+) – ALLE ELEKTRODEN(-) - STROM-REPRINTER
17 - BEIDE ARME(-) – ALLE (+) - STROM-REPRINTER
18 - BEIDE ARME(+) – ALLE (-) - STROM-REPRINTER
19 - BEIDE ARME L(+) – R(-) - STROM-REPRINTER
20 - BEIDE ARME L(-) – R(+) - STROM-REPRINTER
21 - BEIN LINKS(-) – ALLE ELEKTRODEN(+) - STROM-REPRINTER
22 - BEIN LINKS(+) – ALLE ELEKTRODEN(-) - STROM-REPRINTER
23 - BEIN RECHTS(-) – ALLE ELEKTRODEN(+) - STROM-REPRINTER
24 - BEIN RECHTS(+) – ALLE ELEKTRODEN(-) - STROM-REPRINTER
25 - BEIDE BEINE(+) - STROM-REPRINTER
26 - BEIDE BEINE(-) - STROM-REPRINTER
27 - BEIDE BEINE L(-) – R(+) - STROM-REPRINTER
28 - BEIDE BEINE L(+) – R(-) - STROM-REPRINTER
29 - RECHTE SEITE(-) - STROM-REPRINTER
29 - LINKE SEITE(+) - STROM-REPRINTER
30 - RECHTE SEITE(+) - STROM-REPRINTER
30 - LINKE SEITE(-) - STROM-REPRINTER
31 - UNTERE PARTIE(+) - STROM-REPRINTER
32 - UNTERE PARTIE(-)
33 - UNUNTERBROCHENER STROM-REPRINTER (BREITBAND)
NIEDERFREQUENZEN - UNUNTERBROCHENER STROM-REPRINTER
MITTELFREQUENZEN - UNUNTERBROCHENER STROM-REPRINTER
HOCHFREQUENZEN - UNUNTERBROCHENER STROM-REPRINTER
REFERENZFREQUENZEN (15) - FREQUENZENREPRINTER
REFERENZ-NIEDERFREQUENZEN (5) - FREQUENZENREPRINTER
REFERENZ-MITTELFREQUENZEN (5) - FREQUENZENREPRINTER
REFERENZ-HOCHFREQUENZEN (5) - FREQUENZENREPRINTER

Fig. 23

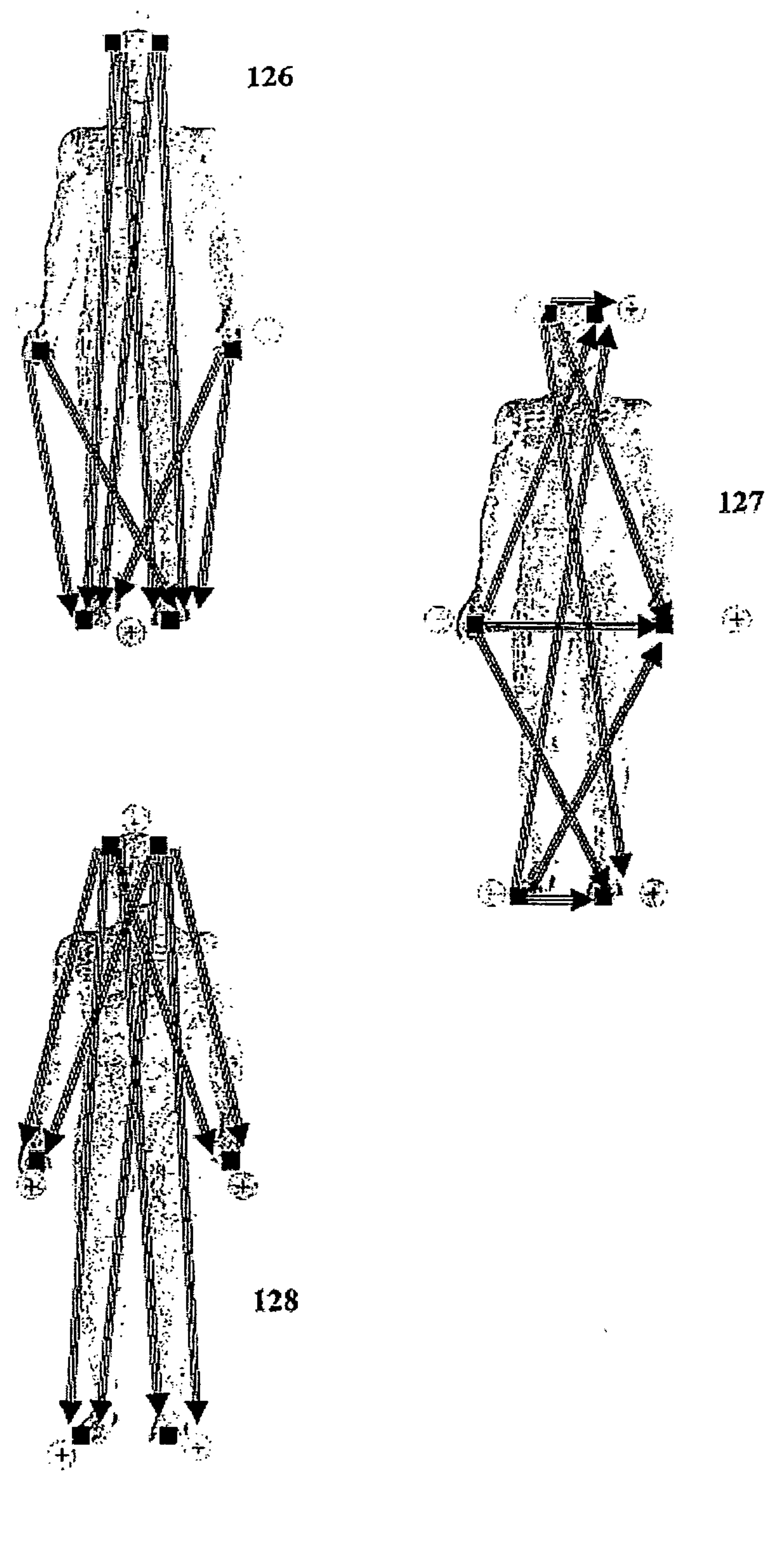


Fig. 24

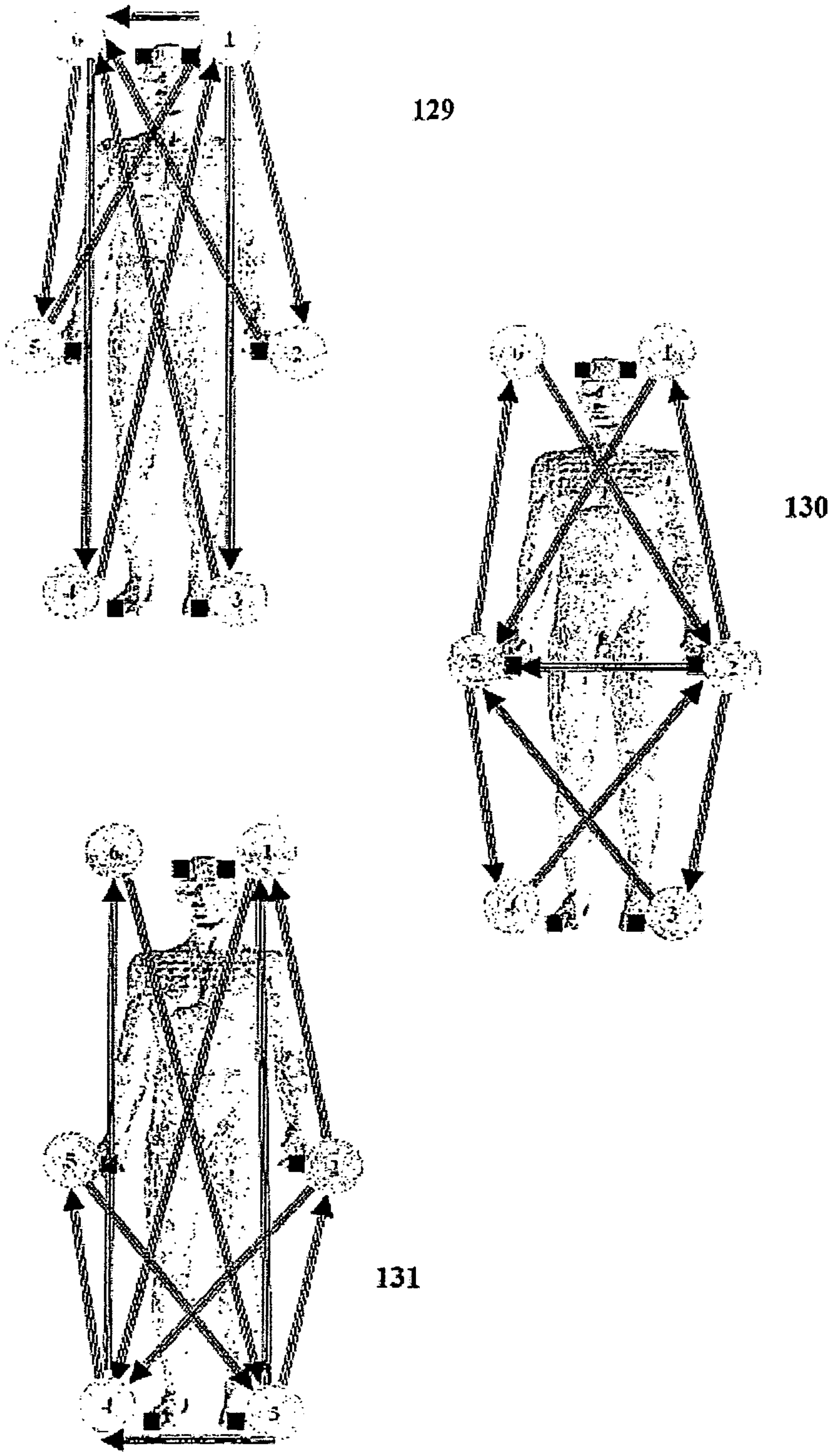


Fig. 25

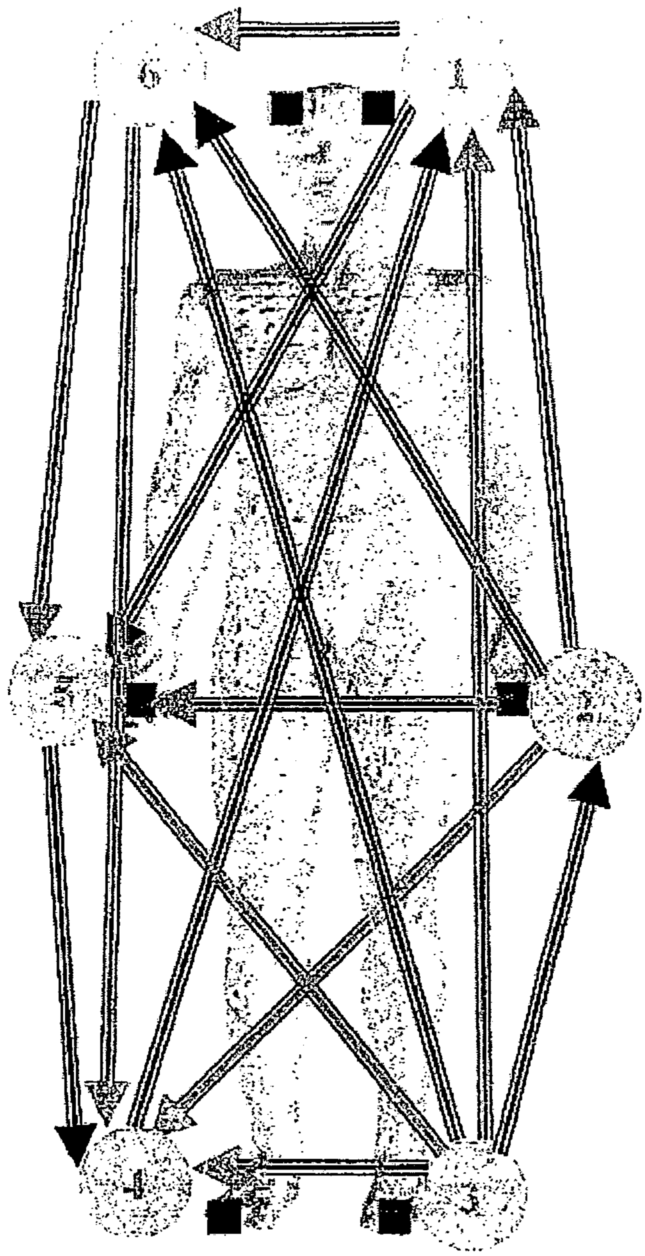

Fig. 26

Abweichung des Eingangs- und Ausgangssignals

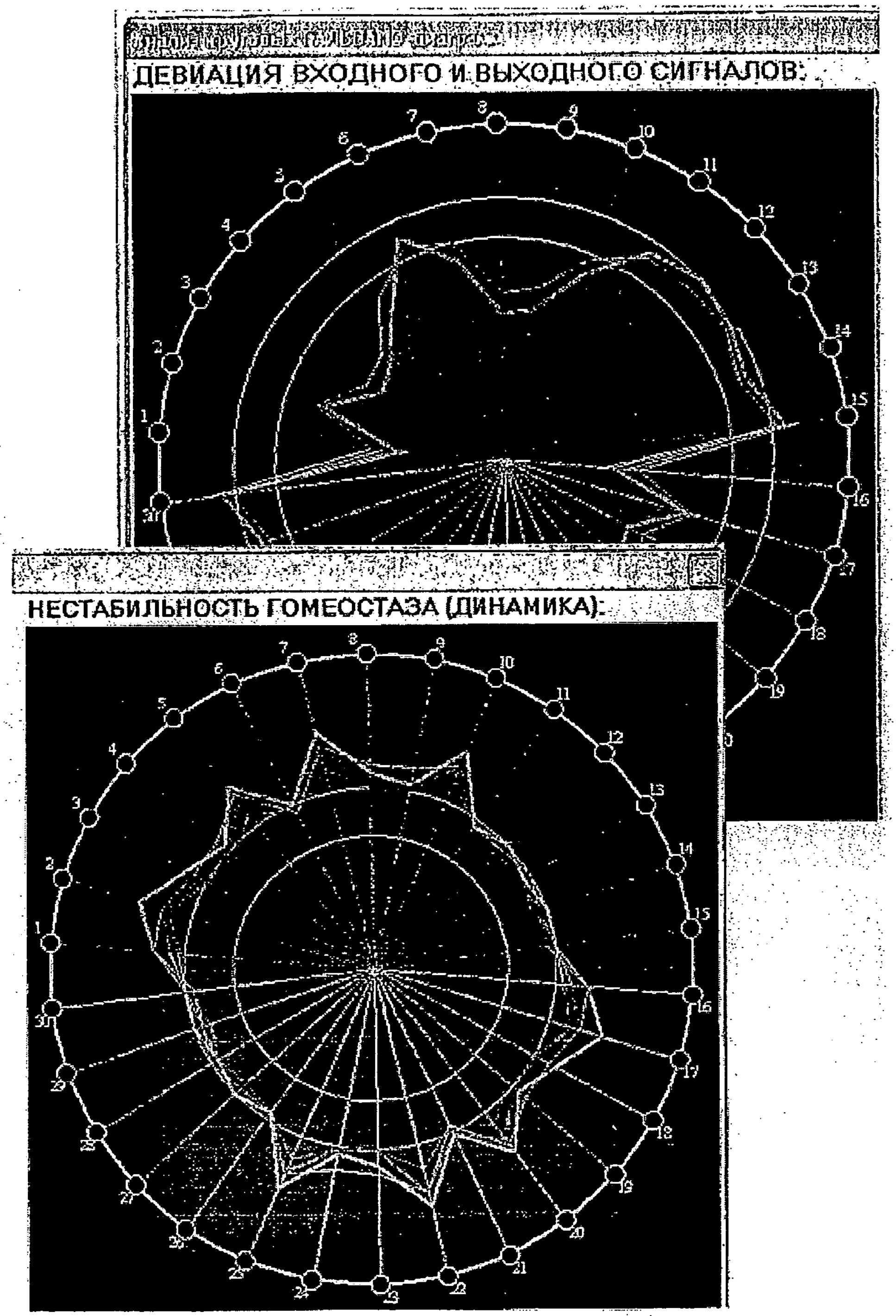


Homöostaseinstabilität (dynamischer Verlauf)

Fig. 27

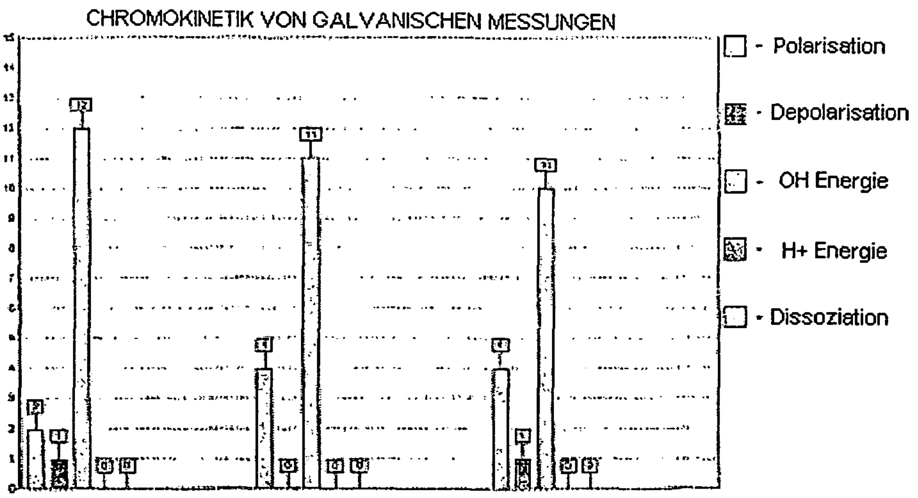
CHROMOKINETIK VON GALVANISCHEN MESSUNGEN
- Polarisation
- Depolarisation
- OH Energie
- H+ Energie
- Dissoziation

Fig. 28

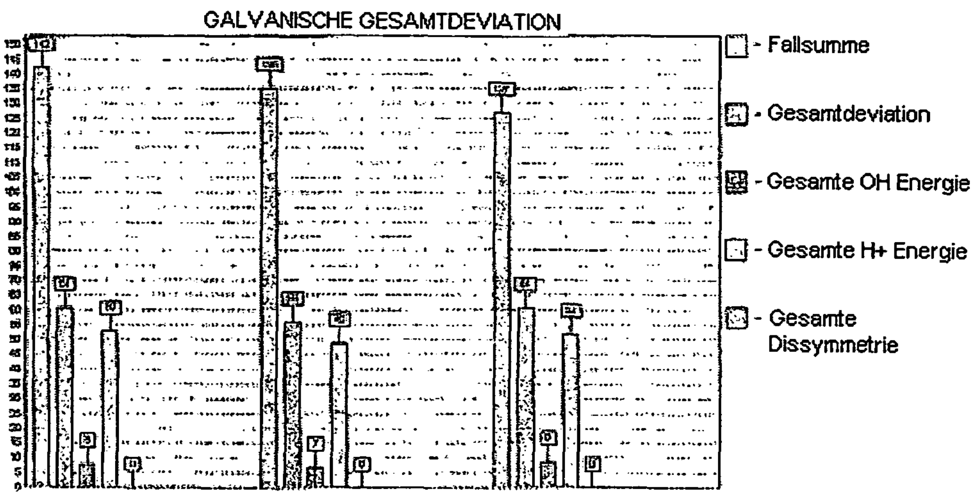
GALVANISCHE GESAMTDEVIATION
- Fallsumme
- Gesamtdeviation
- Gesamte OH Energie
- Gesamte H+ Energie
- Gesamte Dissymmetrie

Fig. 29

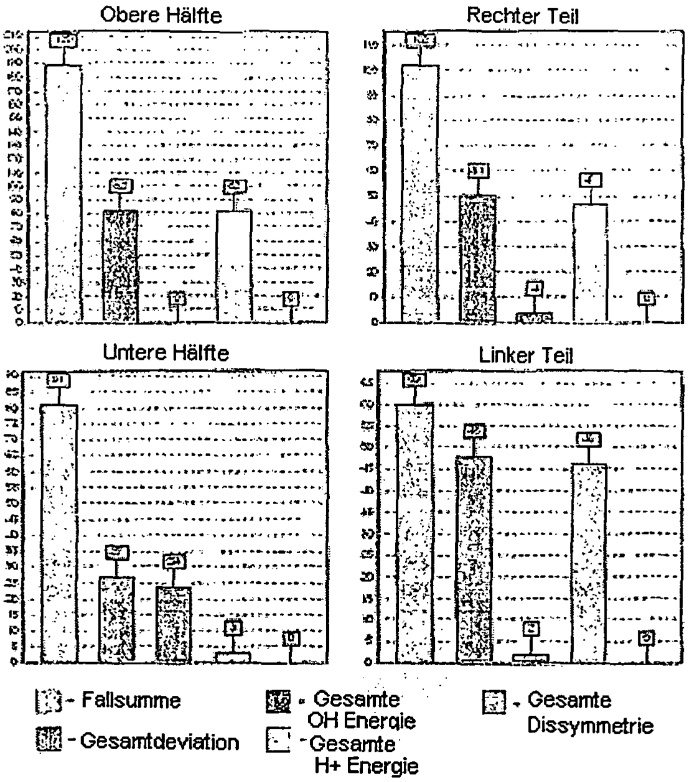


Fig. 30

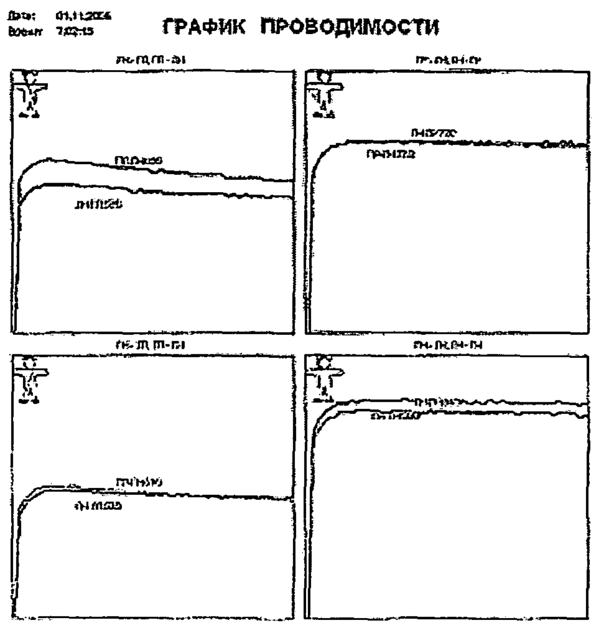


Fig. 31 Leitfähigkeit

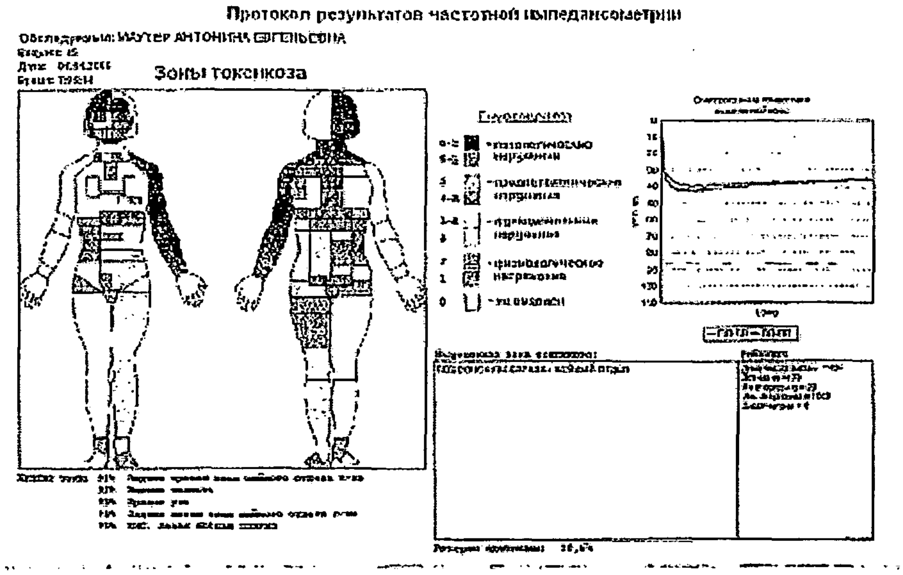


Protokoll der Ergebnisse

Fig. 32

Fig. 17

| [*unleserlich*] | Stufen der Homotoxikose |
|---|---|
| Eventuelle Krankheiten: | Pathologische Mißbildungen / Abweichungen |
| ADNEXEENTFERNUNG LINKS | |
| ANGIOTROPHISCHE NEUROSE VON OBEREN EXTREMITÄTEN | |
| ENDOGENES BRONCHIALASTHMA | Vorpathologische Mißbildungen / Abweichungen |
| NICHTOBSTRUKTIVE BRONCHITIS | |
| OBSTRUKTIVE BRONCHITIS | |
| KATARRALISCHE BRONCHITIS | Funktionelle Mißbildungen / Abweichungen |
| NEUROMYOPATHIE VON OBEREN EXTREMITÄTEN | |
| DISTHYROIDISMUS | |
| HYPOTHYROIDISMUS | Physiologische Belastung |
| HYPERTHYROIDISMUS | |
| ARTHROPATHIE DES RECHTEN ELLENBOGENGELENKES | |
| ARTHROPATHIE DES LINKEN ELLENBOGENGELENKES | Nicht festgestellt |
| ARTHROPATHIE DES RECHTEN HANDGELENKES | |
| ARTHROPATHIE DES LINKEN HANDGELENKES | Anpassungsreserve: 25,7% |
| DIABETISCHE NEUROMYOPATHIE | |
| Empfohlene Arzneimittel: | |
| Phosphor | |
| Cimicifuga | |
| Bryonia | |
| Rumex crispus (Krausblättriger Ampfer) | |
| Scilla | |
| Mutterkorn (?) [*unleserlich*] | |
| Wasserschierling (Cicuta virosa) | |
| Borax (Natriumtetraborat) | Drucken Beenden |

| NAME: VOLKOVA, ELENA NIKOLAEVNA Geschlecht: | W Alter: 22 Benutzer: DOKTOR: |
|---|---|

Fig. 18

| | | *[unleserlich]* | | |
|---|---|---|---|---|
| | 4<br>Hypophysenhinterlappen<br>Lungen<br>Dickdarm<br>Hypophysenmittellappen<br>Tonsille von Torus tubarius<br>Siebbeinhöhlen<br>Nasalhöhlen<br>Handgelenk<br>Ellbogengelenk<br>Schultergelenk<br>Fußgelenk<br>5 | | 6<br>Beschreibung<br>Hypophysenmittellappen<br>Kniegelenk<br>Milz<br>Pankreas<br>Magen<br>Brustdrüsen<br>Obere Nebenschilddrüse<br>Schilddrüse<br>Rachenmandel<br>Kieferhöhle<br>Laryngopharynx<br>Mandibulargelenk (Unterkiefergelenk)<br>7 | Stufen von Homotoxikose<br>Pathologische Mißbildungen / Abweichungen<br>Vorpathologische Mißbildungen / Abweichungen<br>Funktionelle Mißbildungen / Abweichungen<br>Physiologische Belastung<br>Nicht festgestellt<br>Anpassungsreserve: 25.7%<br>Drucken Beenden |
| 8<br>Beschreibung<br>Ohren<br>Handgelenk<br>Ellbogengelenk<br>Schultergelenk<br>Kniegelenk<br>Herz<br>Dünndarm und Zwölffingerdarm<br>Peripheres Nervensystem<br>Zungentonsille<br>Kavernöse Höhle<br>Zunge<br>Iliosakralgelenk | | 1<br>2<br>Beschreibung<br>Blase<br>Prostata<br>Parochis<br>Samenleiter<br>Eierstöcke<br>[unleserlich]<br>Uterus<br>Eileiter<br>Epiphyse<br>Kehlkopfmandel<br>Frontalhöhlen<br>Ohren<br>Wirbelsäule<br>Hüftgelenke<br>Kniegelenke (hinten) | | 3<br>Beschreibung<br>Leber<br>Gallenblase<br>Rachenmandel<br>Sphenoidalhöhlen<br>Augen<br>Muskeln<br>Fußgelenke<br>Kniegelenke (vorne)<br>Eierstöcke<br>[unleserlich]<br>Hoden |

| | |
|---|---|
| NAME: VOLKOVA, ELENA NIKOLAEVNA Geschlecht: | W Alter: 22 Benutzer: DOKTOR: |

(Fig. 19)

DERMATOME – Hautbereiche mit Mikrozirkulationsstörungen. Projektion von dem Ziekorganfantom auf die Körperoberfläche.

Hypophyse

OH(-) HYDRATATION
Stark
Scheinbar
Mittelmäßig
Schwach

NORM

H+ DEHYDRATATION
Schwach
Mittelmäßig
Scheinbar
Stark

Empfindlichkeit 1.3

Drucken Beenden

NAME: SHCHERBINA, LYDIA VIADIMIROVNA Geschlecht: W Alter: 50 Bediener: BUT, Yu.S., Professor, RAEN - OMSK - 3812-24-75-74 - UCHEBA [STUDIES]

(Fig. 20)

DERMATOME – Hautbereiche mit Mikrozirkulationsstörungen. Projektion von dem Ziekorganfantom auf die Körperoberfläche.

Hypophyse

OH(-) HYDRATIQN
Stark
Scheinbar
Mittelmäßig
Schwach

NORM

H+ DEHYDRATATION
Schwach
Mittelmäßig
Scheinbar
Stark

Empfindlichkeit 5.7

Drucken Beenden

GESAMTABWEICHUNG
GESAMT -OH ENERGIE
GESAMT H+ ENERGIE
GESAMT-DISSYMMETRIE

Fig. 32

Protokoll der Ergebnisse von teilweisen Widerstandsmessungen

Subjekt: MAUTER, ANTONINAYEVGENEVNA
Alter: 45
Datum: 11.01.2006
Zeit: 7:05:14 Toxikose-Zonen

Homotoxikose

| | |
|---|---|
| 0-2 | Pathologische Mißbildungen / Abweichungen |
| 5 | Vorpathologische |
| 4-3 | Mißbildungen / Abweichungen |
| 3-4 | Funktionelle |
| 3 | Mißbildungen / Abweichungen |
| 2 | Physiologische |
| 1 | Belastung |
| 0 | Nicht festgestellt |

Homeostasis-Spektrogramm
der markierten Zone

conventional units =
Konventionelle Einheiten
t (ms)

Markierte Toxikose-Zone:
WIRBELSÄULE RECHTS - HALSBEREICH

Bewertung:
[*unleserlich*]
Abweichung 170
[*unleserlich*] Energie = [*unleserlich*]
[*unleserlich*] Energie = [*unleserlich*]
Dissymmetrie - 0

Schlechtere Zonen:
91% Halsbereich in der Zone hinten rechts - an der Wirbelsäule
91% Unterkiefer
91% Rechtes Ohr

INTERNATIONAL SEARCH REPORT

International application No.
PCT/RU 2006/000607

A. CLASSIFICATION OF SUBJECT MATTER
A61B 5/00 (2006.01)
A61B 5/103 (2006.01)
According to International Patent Classification (IPC) or to both national classification and IPC

B. FIELDS SEARCHED

Minimum documentation searched (classification system followed by classification symbols)

A61B 5/00, A61B 5/103

Documentation searched other than minimum documentation to the extent that such documents are included in the fields searched

Electronic data base consulted during the international search (name of data base and, where practicable, search terms used)
PCT Online, USPTO DB, Esp@cenet, DWPI, CIPO (Canada PO), SIPO DB, AIPN, DEPATISnet, NCBI PubMed , VINITI.RU, SCSML.FSSI.RU

C. DOCUMENTS CONSIDERED TO BE RELEVANT

| Category* | Citation of document, with indication, where appropriate, of the relevant passages | Relevant to claim No. |
|---|---|---|
| A | US 2003/0195427 A1 (LEONID VASILYEVICH MASAKOV et al.) 16.10.2003 | 1-4 |
| A | US 6228033 B1 (TIIT KOOBI et al.) 08.05.2001 | 1-4 |
| A | RU 2231974 C2 (OBSCHESTVO S OGRANICHENNOI OTVETSTVENNOSTIJU "TSENTR NOVYKH TEKHNOLOGY") 10.07.2004 | 1-4 |
| A | RU 2129420 C1 (OBSCHESTVO S OGRANICHENNOI OTVETSTVENNOSTIJU MEDIKO-TEKHNICHESKY TSENTR "KOBERT") 27.04.1999 | 1-4 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 July 2007 | 09 August 2007 |
| Name and mailing address of the ISA/<br>RU<br><br>Facsimile No. | Authorized officer<br><br>Telephone No. |

Form PCT/ISA/210 (second sheet) (April 2005)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **Pflaum, H.** Praktikum der bioelektronischen Funktionen und der Regulationsdiagnostik (BFD). HF. Heidelberg, 1979, 1-137
- **Moskau.** *Thesen und Berichte der V. Internationalkonferenz für BRT,* 1999, 380